# EUROPEAN PATENT SPECIFICATION

(11) **EP 3 413 888 B1**
(45) Date of publication and mention of the grant of the patent: **10.05.2023**
(21) Application number: 17750983.3
(22) Date of filing: 13.02.2017
(51) Int. Cl.: A61K 31/27, A61K 31/40, A61N 1/30, A61M 31/00, A61P 1/00

(54) **BOWEL CARE USING IONTOPHORESIS**
DARMPFLEGE MITHILFE VON IONTOPHORESE
SOIN DE L'INTESTIN PAR IONTOPHORÈSE

(30) Priority: 12.02.2016 US 201662294874 P
(43) Date of publication of application: 19.12.2018
(73) Proprietor: United States Government as Represented by The Department of Veterans Affairs, Washington, DC 20420 (US)
(72) Inventor: KORSTEN, Mark A., Washington, District of Columbia 20420 (US); BAUMAN, William A., Washington, District of Columbia 20420 (US)
(74) Representative: Viering, Jentschura & Partner mbB Patent- und Rechtsanwälte
(86) International application number: PCT/US2017/017717
(87) International publication number: WO 2017/139794

(56) References cited:
- WO-A1-01/08681
- WO-A2-01/35927
- WO-A2-2005/074535
- US-A- 4 722 726
- US-A- 5 688 232
- US-A1- 2004 082 644
- US-A1- 2004 082 644
- US-A1- 2006 013 866
- US-A1- 2006 013 866
- US-A1- 2009 253 634
- US-A1- 2013 172 398
- US-A1- 2014 207 047
- US-A1- 2015 297 718

## Description

### BACKGROUND

### Field of the Invention

The present disclosure generally relates to compositions for use in methods of treatment of chronic intestinal pseudo-obstruction and more specifically relates to combinations ionic of acetylcholinesterase inhibitors, such as neostigmine, with ionic anti-cholinergic agents, such as glycopyrrolate and delivery of such drug combinations by iontophoresis for on-going bowel care in individuals with chronic intestinal pseudo-obstruction, such as persons with spinal cord injuries.

### Related Art

Normal movement of stool through the colon depends on periodic contractions of the muscles in the wall of the colon that push (or squeeze) fecal matter in the direction of the rectum, which ultimately results in evacuation of the feces. These contractions normally are stimulated by nerves arising from the lower or sacral part of the spinal cord (i.e., S2-4). The effects of these nerves on the colon are generally mediated by a substance called acetylcholine, which causes muscles in the wall of the colon to contract.

The neural control mechanisms of the gastrointestinal (GI) tract are thought to be (or to become) impaired, at least in part, in a number of diseases and medical conditions, e.g., spinal cord injury, amyotrophic lateral sclerosis, spina bifida, multiple sclerosis, Parkinson's disease and dementias, even though the colonic muscles remain intact and capable of responding to acetylcholine. As a result, persons afflicted with these conditions often experience difficulty with bowel functions, including the inability to initiate defecation, straining to defecate, or incomplete evacuation of feces. Because of the chronic nature of the underlying disease, the resulting bowel dysfunction is also chronic and may have a significant negative impact on the subject's quality of life.

Traditional approaches to bowel care for individuals with chronic bowel dysfunction typically involve the periodic administration of laxatives and enemas in combination with digital stimulation of the rectum. These approaches are time consuming and expensive, have unpredictable efficacy, and may cause damage to the bowel. For example, incomplete emptying of the bowel at the time of bowel care increases the likelihood of incontinence, i.e., "accidents." Moreover, the physical trauma of bowel care procedures leads to an increased risk of anorectal problems, especially bleeding hemorrhoids and anal fissures.

Neostigmine is a drug that has long been used by anesthesiologists to reverse the muscle paralysis artificially induced during surgical procedures. Neostigmine is a reversible acetylcholinesterase inhibitor, which blocks the breakdown of the neurotransmitter acetylcholine. This results in an accumulation of acetylcholine in synaptic spaces, which causes (among other things) contractions of the smooth muscles of the bowel.

While smooth muscle contractions in the gut were considered an unwanted side effect by anesthesiologists, other clinicians (e.g., Ponec et al., New Engl. J. Med., 341(3):137-141, 1999) have exploited this neostigmine effect in the treatment of acute colonic pseudo-obstruction. Acute colonic pseudo-obstruction is a relatively rapid onset, intense, short-term condition characterized by bowel distension, constipation, abdominal pain, and the absence of an actual mechanical blockage. Following neostigmine administration for this condition, most patients successfully passed flatus or stool with a corresponding reduction in abdominal distention. However, as expressly recognized by Ponec et al., neostigmine treatment is not without risk. Known side effects of neostigmine include bradycardia (i.e., slowing of the heart rate), excessive pharyngeal and laryngeal secretions, nausea, vomiting, abdominal cramps, and diarrhea. In particular, neostigmine-induced bradycardia can become life threatening and requires close cardiac monitoring in a clinical setting.

Glycopyrrolate is an anti-cholinergic agent (more particularly, a muscarinic antagonist), which blocks neurotransmission by acetylcholine. Glycopyrrolate (or other anti-cholinergic agents, such as atropine) are used by anesthesiologists in the operating room setting or by other clinicians in clinical settings and essentially on emergency basis to counteract the cardiac (e.g., bradycardia) and pulmonary (e.g., bronchoconstriction) side effects caused by neostigmine administration.

The potentially life-threatening side effects of neostigmine have historically restricted its usefulness to the clinical setting where the drug recipient is carefully monitored. Even though medications, such as glycopyrrolate, are available to counteract the side effects of neostigmine, those medications have traditionally been administered non-repetitively in the clinical setting only at the time the side effects of neostigmine are induced. For all of these reasons, the routine use of neostigmine for the treatment of a chronic medical condition in a non-clinical setting has been considered unacceptable.

A recent solution for bowel care for individuals with chronic bowel dysfunction is a combination of an acetylcholinesterase inhibitor, such as neostigmine, and an anti-cholinergic agent, such as glycopyrrolate. This combination may be safely administered in repeated doses over a period of time in an ongoing bowel care program for subjects with chronic intestinal pseudo-obstruction. However, administration of the drug combination to an individual is expensive, uncomfortable, time consuming and can be very difficult to administer to individuals with chronic bowel dysfunction caused by spinal cord injuries.

Intravenous administration of the drug combination, although highly effective, is impractical for ongoing at home bowel care because it requires a trained health professional for drug administration. Intramuscular administration is also highly effective, but impractical for ongoing at home bowel care because it requires a trained health professional for drug administration. Intramuscular administration also results in local muscle pain and soreness. Subcutaneous administration suffers from too gradual adsorption and consequently fails to induce bowel evacuation. Oral or sublingual administration similarly suffers from a slower and less reliable rate of absorption. Intranasal administration of the drug combination also fails to induce bowel evacuation. Accordingly, only parenteral intravenous or intramuscular delivery (i.e., via needle) has proven successful for inducing bowel evacuation and such administration suffers from serious drawbacks for ongoing at home bowel care because it could result in autonomic dysreflexia, a potentially life-threatening condition and because it requires a trained health professional for drug administration.

U.S. Patent Application No. 2004/0082644 A1 discloses compositions and methods for on-going bowel care for persons with chronic intestinal pseudo-obstruction suitable for administration in a non-clinical setting. The methods comprise the repeatedly performed administration of compositions comprising the acetylcholinesterase inhibitor neostigmine and the anti-cholinergic agent glycopyrrolate. Methods of administration include intravenous and intramuscular injection as well as in the form of rectal suppositories.

Thus, there remains a need for improved at home bowel care treatment for individuals with spinal cord injury and other medical conditions and disorders.

### SUMMARY

A combination of an acetylcholinesterase inhibitor, such as neostigmine, and an anti-cholinergic agent, such as glycopyrrolate, may be safely administered in repeated doses over a period of time as part of an ongoing bowel care program for subjects with chronic intestinal pseudo-obstruction. The present disclosure provides systems and methods for routine, self administered in-home bowel care for persons with chronic intestinal pseudo-obstruction. The compositions can be administered using iontophoresis on a regular basis in a home setting without the need of a trained health professional. The compositions comprise acetylcholinesterase inhibitors for stimulating motility of the bowel in combination with anti-cholinergic agents to counteract the potentially dangerous cardiopulmonary side effects of the acetylcholinesterase inhibitor. According to the invention, the acetylcholinesterase inhibitor, neostigmine, and the anti-cholinergic agent, glycopyrrolate, are combined in a pharmaceutical composition. The described methods provide for administration of the disclosed drug combinations as desired to implement in-home bowel care.

Certain embodiments of the disclosed methods and compositions can provide advantages such as (i) avoiding incomplete evacuation of the bowels, which may lead to incontinence, (ii) avoiding physical trauma of traditional bowel care methods, and thus reduce the risk of ano-rectal problems such as bleeding hemorrhoids and anal fissures, (iii) providing a more comfortable method of administration, and (iv) providing a relatively rapid therapeutic response.

Disclosed herein is further a system comprising an iontophoresis apparatus having a memory, a processor, a power source, one or more electrical leads and one or more electrodes, each connected to an electrical lead. The system also includes one or more iontophoresis patches comprising a combination of an acetylcholinesterase inhibitor and an anti-cholinergic agent, for example, neostigmine and glycopyrrolate. In one embodiment, a first patch includes the acetylcholinesterase inhibitor and a second patch includes the anti-cholinergic agent. In an alternative embodiment, the acetylcholinesterase inhibitor and the anti-cholinergic agent are combined on a single patch. The one or more iontophoresis patches are configured to contact the skin of the user and the electrodes are configured to deliver a current to the skin of the user at the location of the one or more iontophoresis patches. The inventors have surprisingly discovered that iontophoresis provides an effective administration of both neostigmine and glycopyrrolate at the desired dosage to promote timely bowel evacuation for at-home self administered bowel care.

Other features and advantages of the present invention will become more readily apparent to those of ordinary skill in the art after reviewing the following detailed description and accompanying drawings.

### BRIEF DESCRIPTION OF THE DRAWINGS

The structure and operation of the present invention will be understood from a review of the following detailed description and the accompanying drawings in which like reference numerals refer to like parts and in which:
FIG. 1 shows digital video fluoroscopy images (panels A-E) of the colons of paraplegic or quadriplegic subjects who had received rectal instillation of a barium-oatmeal paste followed by intravenous administration of a combination of 2 mg neostigmine and 0.4 mg glycopyrrolate. Each of these subjects was having difficulty with evacuation (DWE) at the time of study. The images were collected after a complete response to the drug combination. Panels A, B, C, D, and E represent responses that were adjudged to be 0, 1+, 2+, 3+ and 4+ bowel evacuation scores, respectively.
FIG. 2 shows a graph of the bowel evacuation scores of thirteen paraplegic or quadriplegic subjects with DWE who received an intravenous bolus of either 2 mg neostigmine alone (black bar) or normal saline (stippled bar).
FIG. 3 shows a graph of the bowel evacuation scores of thirteen paraplegic or quadriplegic subjects with DWE who received an intravenous bolus of either a combination of 2 mg neostigmine and 0.4 mg glycopyrrolate (cross-hatched bar) or normal saline (stippled bar).
FIG. 4 shows a graph of the bowel evacuation scores of thirteen paraplegic or quadriplegic subjects with DWE who received an intravenous bolus of either 2 mg neostigmine alone (black bar) or a combination of 2 mg neostigmine and 0.4 mg glycopyrrolate (cross-hatched bar).
FIG. 5 shows a graph of the percentage of paraplegic or quadriplegic subjects (13 total) having a bowel evacuation score greater than 3 after receiving an intravenous bolus of either 2 mg neostigmine alone (black bar) or a combination of 2 mg neostigmine and 0.4 mg glycopyrrolate (cross-hatched bar).
FIG. 6 shows a graph of the time course of the heart rate of thirteen paraplegic or quadriplegic subjects with DWE who received an intravenous bolus of either 2 mg neostigmine alone (black line) or a combination of 2 mg neostigmine and 0.4 mg glycopyrrolate (grey line).
FIG. 7 shows a graph of the lowest heart rate measured in thirteen paraplegic or quadriplegic subjects with DWE who received an intravenous bolus of either 2 mg neostigmine alone (black bar) or a combination of 2 mg neostigmine and 0.4 mg glycopyrrolate (cross-hatched bar).
FIG. 8 shows a graph of the effects of 2 mg neostigmine (black bar) and a combination of 2 mg neostigmine and 0.4 mg glycopyrrolate (cross-hatched bar) on total airway resistance in thirteen paraplegic or quadriplegic subjects with DWE as measured by the forced oscillation technique using 5 Hz oscillations.
FIG. 9 shows a graph of the effects of 2 mg neostigmine (black bar) and a combination of 2 mg neostigmine and 0.4 mg glycopyrrolate (cross-hatched bar) on central airway resistance in thirteen paraplegic or quadriplegic subjects with DWE as measured by the forced oscillation technique using 20 Hz oscillations.
FIG. 10 is a block diagram illustrating an example processor enabled wired or wireless system 550 that may be used in connection with various embodiments described herein.

### DETAILED DESCRIPTION

### I. Introduction

Any references to method of treatment are to be construed as referring to a use in a method of treatment. This specification discloses methods of bowel care for subjects having chronic intestinal pseudo-obstruction. Such methods include chronically administering to the subject a therapeutically effective amount of a drug combination including an acetylcholinesterase inhibitor and an anti-cholinergic agent. In particular embodiments, the chronic administration occurs at least one time per week or at least three times per week over a period of at least one month or over a period of at least six months.

According to the invention, the acetylcholinesterase inhibitor is neostigmine and the anti-cholinergic agent is glycopyrrolate.

In some methods, a therapeutically effective amount of the drug combination is about 1 mg to about 2 mg neostigmine and about 0.2 mg to about 0.4 mg glycopyrrolate. According to the invention, the therapeutically effective amount of the drug combination is a ratio of neostigmine to glycopyrrolate of about 2.5:1 to about 10:1 by weight, or in more particular methods, of about 5:1 by weight.

In some methods, the intestinal pseudo-obstruction is an effect of spinal cord injury, amyotrophic lateral sclerosis, spina bifida, multiple sclerosis, Parkinson's disease or dementia. In particular methods, the intestinal pseudo-obstruction is an effect of spinal cord injury, such as paraplegia or quadriplegia.

In particular examples of the disclosed methods, the acetylcholinesterase inhibitor and the anti-cholinergic agent are administered at about the same time. In other methods, the anti-cholinergic agent is administered after the acetylcholinesterase inhibitor, for example about 1 to about 10 minutes after the acetylcholinesterase inhibitor.

The method of administration of the acetylcholinesterase inhibitor or the anti-cholinergic agent is iontophoresis, for example using an iontophoresis delivery device such as a Dynatron iBox^{™}. Also disclosed herein are methods of bowel care for a subject with chronic intestinal pseudo-obstruction, which includes identifying a subject having chronic intestinal pseudo-obstruction as an effect of spinal cord injury; co-administering to the subject a therapeutically effective amount of a drug combination comprising neostigmine and glycopyrrolate in a 5:1 ratio by weight. In particular embodiments, the drug combination is periodically administered, for example, at least one time per week and up to once per day or more, for at least one month or longer, for example at least six months.

According to the invention, pharmaceutical compositions include a therapeutically effective amount of a combination of neostigmine and glycopyrrolate in a weight ratio of neostigmine to glycopyrrolate of 2.5:1 to 10:1. In certain embodiments, the weight ratio of neostigmine to glycopyrrolate is about 5:1. In other embodiments, the pharmaceutical composition includes about 1 mg to about 2 mg neostigmine and about 0.2 mg to about 0.4 mg glycopyrrolate, or more specifically about 2 mg neostigmine and about 0.4 mg glycopyrrolate.

### Abbreviations:

BMP: beats per minute
DWE: difficulty with evacuation
GI: gastrointestinal
HR: heart rate
IM: intramuscular(ly)
IV: intravenous(ly)
SCI: spinal cord injury
SI: spinally intact

### III. Terms

Unless otherwise noted, technical terms are used according to conventional usage. In order to facilitate review of the various embodiments of the invention, the following explanations of specific terms are provided:
Acetylcholine: An acetic acid ester of choline, which serves as a neurotransmitter at the myoneural junctions of striated muscles, at autonomic effector cells innervated by parasympathetic nerves, at the preganglionic synapses of the sympathetic and parasympathetic nervous systems, and at various sites in the central nervous system.

Acetylcholinesterase: An enzyme that catalyzes the cleavage of acetylcholine to choline and acetate. The action of acetylcholinesterase in the synaptic cleft suppresses the neurotransmitter effect of acetylcholine on the post-synaptic cell.

Acetylcholinesterase inhibitor: An agent that interferes with the enzymatic activity of acetylcholinesterase, which results, for example, in local accumulations of acetylcholine in synapses where acetylcholine serves as a neurotransmitter. Representative acetylcholinesterase inhibitors are neostigmine, physostigmine, ambenonium, pyridostigmine, edrophonium, demecarium, echothiophate, or pralidoxime.

Agent: Any substance, including, but not limited to, an antibody, chemical compound, molecule, peptidomimetic, or protein.

Anti-cholinergic agent: Agents that suppress the effects of acetylcholine on the nervous system. Drugs with anti-cholinergic effects inhibit the secretion of acid in the stomach, slow the passage of food through the digestive system, inhibit the production of saliva, sweat, and bronchial secretions, and increase the heart rate and blood pressure. Adverse effects of these drugs include dry mouth, constipation, difficulty urinating, confusion, worsening of glaucoma, blurred vision, and short-term memory problems. Representative anti-cholinergic agents include, for example, anisotropine, atropine, clidinium, dicyclomine, glycopyrrolate, hexocyclium, homatropine, hyoscyamine, ipratropium, isopropamide, mepenzolate, methantheline, methscopolamine, oxyphencyclimine, oxyphenonium, pirenzepine, propantheline, scopolamine, or tridihexethyl.

Bradycardia: A term generally used to mean slowing of the heart rate. Bradycardia may be defined in terms of absolute or relative numbers of heart beats per minute (BPM) or in symptomatic terms. As used herein, "absolute bradycardia" means slowing of the heart rate to less than a specified number of BPM; for example, the slowing of the heart rate to less than about 60 BPM, or to less than about 58 BPM, or to less than about 55 BPM, or to less than about 52 BPM, or to less than about 50 BPM, or to less than about 48 BPM. "Relative bradycardia" means that the baseline heart rate of a particular subject has decreased by a specified number of BPM, or by a specified percentage. The "baseline heart rate" is the heart rate of a subject prior to a perturbation in the subject that affects the heart rate; for example, the heart rate of a subject prior to the administration of an acetylcholinesterase inhibitor, such as, neostigmine, which effects a slowing of the heart rate. In some examples of relative bradycardia, the heart rate in a subject may decreased about 5 BPM below baseline heart rate, or about 7 BPM below baseline heart rate, or about 10 BPM below baseline heart rate, or about 12 BPM below baseline heart rate, or about 15 BPM below baseline heart rate. In other examples of relative bradycardia, the baseline heart rate in a subject may decrease by about 5%, or by about 7%, or by about 10%, or by about 12%, or by about 15%, or by about 20%, or by about 25%, or by about 30%, or by about 35%, or by about 40%, or by about 50%. As used herein, the term "symptomatic bradycardia" is not limited to any specific heart rate or decrease in heart rate; instead, symptomatic bradycardia is describes a slowed heart rate that is accompanied by hypotension (e.g., systolic blood pressure drops by 25% or more), dizziness, extreme fatigue, shortness of breath, and/or fainting spells (i.e., syncope). In some specific examples of symptomatic bradycardia, a subject experiences only hypotension. In some specific examples of symptomatic bradycardia, a subject experiences only dizziness. In other examples of symptomatic bradycardia, a subject experiences hypotension and dizziness. In other examples of symptomatic bradycardia, a subject experiences dizziness and fainting. In still other examples of symptomatic bradycardia, a subject experiences dizziness and shortness of breath. As used herein, "substantial bradycardia" is bradycardia in which the heart rate slows to less than 45 BPM, or in which the heart rate in a subject decreases by 20 BPM below baseline heart rate, or in which the baseline heart rate in a subject decreases by 25% or in which no symptoms of symptomatic bradycardia are observed.

Chronically administering [a drug combination]: The prolonged administration of separate doses of a drug combination to a subject over a period of time. The prolonged administration can occur at regular prescribed intervals or at irregularly intervals (for example, as needed). For example, a chronically administered drug combination may be administered at least twice daily, at least daily, at least five times per week, at least three times per week, at least two times per week, at least one time per week, or at least once every 10 days for at least two administrations, at least 5 administrations, at least 10 administrations, at least 25 administrations, at least 50 administrations, at least 100 administrations, or at least 500 administrations. Accordingly, chronic administration of a drug combination may be continued for weeks, months or years; for example, at least two weeks, at least one month, at least two months, at least three months, at least six months, at least one year, at least five years, or a lifetime of a subject. In particular embodiments of the disclosed methods, a combination of an acetylcholinesterase inhibitor (such as, neostigmine) and an anti-cholinergic agent (such as, glycopyrrolate) are chronically administered at least daily for at least two weeks, or at least three times per week for at least one month, or at least three times per week for at least six months, or at least three times per week for one year, or at least three times per week for a subject's lifetime, or at least five times per week for a subject's lifetime.

Co-administering or co-administration: Administration of two or more agents at substantially the same time in the same or separate dosage forms.

Dementia: An acquired organic mental disorder with loss of intellectual abilities of sufficient severity to interfere with social or occupational functioning. The dysfunction involves, for example, memory, behavior, personality, judgment, attention, spatial relations, language, abstract thought, and other executive functions. The intellectual decline is usually progressive, and initially spares the level of consciousness. Representative dementias include, for example, multi-infarct dementia (as an effect of, for instance, multiple strokes) and Alzheimer disease.

Difficulty with evacuation (or DWE): The inability to initiate defecation, straining to defecate, or incomplete evacuation of fecal matter.

Dosage form: The physical form of a pharmaceutical preparation, which contains amounts of one or more medicaments and, generally, but not necessarily, one or more other ingredients. Dosage forms include, for example, injectables, capsules, liniments, ointments, solutions, powders, tablets, suppositories, transnasal sprays, and sublingual tablets or drops.

Drug combination: A physical and/or functional combination of two or more drugs. The drugs of a drug combination may be included together in a single dosage form, such as in a rectal suppository, an injectable, a transnasal spray, a sublingual tablet or drops, an oral formulation, or a transdermal patch; in which case, the drug combination would most often be administered simultaneously. For example, a drug combination comprising an acetylcholinesterase inhibitor and an anti-cholinergic agent may be combined in a single injectable, which is administered by intravenous or intramuscular injection. Alternatively, the drugs of a drug combination may be provided in different or separate dosage forms, such as in two separate injectables, or in a rectal suppository and an injectable, or in a rectal suppository and a sublingual tablet. Any combination of dosage forms is contemplated as long as the drugs of the drug combination are administered in a manner and at a time that permits the drugs of the combination to treat the chronic intestinal pseudo-obstruction without substantial bradycardia, for example without symptomatic bradycardia. For example, a drug combination comprising an acetylcholinesterase inhibitor and an anti-cholinergic agent may be administered by first injecting the acetylcholinesterase inhibitor intravenously (or intramuscularly) and then, after about 1 to 10 minutes, injecting the anti-cholinergic agent intravenously (or intramuscularly).

Glycopyrrolate (also known as glycopyrronium): An agent that belongs to the class of drugs called anti-cholinergic agents, or more specifically to the class of drugs known as muscarinic antagonists, or even more specifically to the class of drugs known as synthetic, quaternary muscarinic antagonists. The chemical name of one form of glycopyrrolate is pyrrolidinium 3-((cyclopentylhydroxyphenylacetyl)oxy) 1, 1-dimethyl-bromide.

Injectable composition: A pharmaceutically acceptable fluid composition comprising at least one active ingredient, for example, an acetylcholinesterase inhibitor (such as, neostigmine), an anti-cholinergic agent (such as, glycopyrrolate) or a combination of an acetylcholinesterase inhibitor and an anti-cholinergic agent (such as, a combination of neostigmine and glycopyrrolate). The active ingredient is usually dissolved or suspended in a physiologically acceptable carrier, and the composition may additionally comprise one or more non-toxic auxiliary substances, such as emulsifying agents, preservatives, pH buffering agents and the like. Such injectable compositions, which may be used with certain embodiments of the drug combinations described herein, are conventional and appropriate formulations are well known in the art.

Intestinal pseudo-obstruction (or pseudo-obstruction): A condition characterized by signs and symptoms that are usually indicative of intestinal obstruction, such as bowel distension, constipation and abdominal pain, but which occur in the absence of an actual mechanical blockage. "Acute intestinal pseudo-obstruction" is a relatively rapid onset, intense, short-term occurrence of intestinal pseudo-obstruction. "Chronic intestinal psuedo-obstruction" is persistent, recurring intestinal pseudo-obstruction, such as occurs, for example, as a result of spinal cord injury, amyotrophic lateral sclerosis, spina bifida, or multiple sclerosis. Unlike the acute condition, chronic intestinal pseudo-obstruction persistently recurs even after a single bowel evacuation treatment, for example, treatment with the combination of an acetylcholinesterase inhibitor (such as neostigmine) and an anti-cholinergic agent (such as glycopyrrolate). In particular examples, chronic intestinal pseudo-obstruction persists for at least two weeks, and in many instances persists for at least one month, at least six months, at least one year, or even for a lifetime.

Muscarinic antagonist: A class of drugs that bind to, but do not activate, muscarinic cholinergic receptors, and thereby block the actions of endogenous acetycholine or exogenous agonists. Muscarinic antagonists are a class of anti-cholinergic drugs. Muscarinic antagonists can have widespread effects including actions on the iris and ciliary muscle of the eye, the heart and blood vessels, secretions of the respiratory tract, Gl system, and salivary glands, GI motility, urinary bladder tone, and the central nervous system. Representative muscarinic antagonists include, for example, anisotropine, atropine, belladonna, clidinium dicyclomine, glycopyrrolate, homatropine, hyoscyamine, hyoscyamine, isopropamide, mepenzolate, methantheline, methscopolamine, oxyphencyclimine, pirenzepine, propantheline, scopolamine, and tridihexethyl.

Neostigmine: A drug that belongs to the broader class of agents called acetylcholinesterase inhibitors. One form of the drug, commonly called neostigmine bromide, has the chemical name (m-hydroxyphenyl)trimethylammonium bromide dimethylcarbamate. Another form of the drug, commonly called neostigmine methyl sulfate, has the chemical name (m-hydroxyphenyl) trimethylammonium methyl sulfate dimethylcarbamate.

Parenteral: Administered outside of the intestine, e.g., not via the alimentary tract. Generally, parenteral formulations are those that will be administered through any possible mode except ingestion. This term refers, for example, to injections, whether administered intravenously, intrathecally, intramuscularly, intraperitoneally, or subcutaneously, rectal suppositories, sublingual drops or tablets, and various surface applications including intranasal, intradermal, and topical application. Parenteral administration is preferred for some drugs to avoid degradation of the drug in the gastrointestinal tract, and/or to provide rapid and/or localized absorption or application.

Pharmaceutical agent (or drug): A chemical compound or other composition capable of inducing a desired therapeutic or prophylactic effect when properly administered to a subject.

Spinal cord injury: Any injury of the neural elements within the spinal canal. Spinal cord injury can result from either trauma (e.g., contusion, compression or laceration) or disease to the vertebral column or the spinal cord itself. Most spinal cord injuries are the result of trauma to the vertebral column. These injuries can affect the spinal cord's ability to send and receive messages from the brain to the body systems that control sensory, motor, and autonomic function below the level of injury.

Spinal cord injury may be classified "complete," when the nerve damage obstructs every signal coming from the brain to body parts below the injury, or "incomplete," when only some of the signals are obstructed. In an incomplete injury, the amount and type of message that can pass between the brain and parts of the body will depend on how many nerves have not been damaged. The vertebral level at which the spinal cord injury occurs is often used as a reference point. The closer the injury is to the brain, the greater the loss of function will be.

Persons with paraplegia or quadriplegia are examples of persons with spinal cord injury. A person is said to have paraplegia when he or she has lost feeling and is not able to move the lower parts of the body. Someone with quadriplegia (or, tetraplegia) has lost movement and feeling in both the upper and lower parts of the body.

Subject: Living multicellular vertebrate organisms, a category which includes both human and veterinary subjects for example, mammals, rodents, and birds.

Therapeutically effective amount of a drug combination: The quantity of each of specified agents in a specified drug combination that is sufficient to achieve a desired effect in a subject being treated. For example, this may be the amount of an acetylcholinesterase inhibitor (such as, neostigmine) and the amount of an anti-cholinergic agent (such as, glycopyrrolate) that when combined are sufficient to induce an episode of bowel evacuation with no substantial bradycardia (whether absolute, relative or symptomatic bradycardia) in a subject with chronic intestinal pseudo-obstruction. Specific drug combinations useful for treating chronic intestinal pseudo-obstruction are described herein. Ideally, a therapeutically effective amount of a drug combination includes the amounts of the combined drugs sufficient to induce one episode of bowel evacuation without causing bradycardia or other substantial side effect in the subject being treated. However, the effective amount of a drug combination described herein will be dependent on the subject being treated, the severity of the affliction, and the manner of administration of the therapeutic composition.

An effective amount of a drug combination is intended to be administered so as to induce a single bowel evacuation event; however, it is contemplated that more than one dose may be given to prompt a single bowel evacuation as needed. Regularly occurring doses, for example, at at least twice daily, at least daily, at least three times per week, at least two times per week, at least one time per week, at least once every 10 days, at least once every 14 days, or at least once every 20 days are contemplated as needed for bowel care in a particular circumstance. However, the frequency of administration will be dependent on the preparation applied, the subject being treated, the severity and type of the affliction, and the manner of administration of the compound.

Transdermal formulation: A formulation of a drug or drug combination that promotes the absorption of the drug(s) from the skin (also called a skin patch).

Waste disposal repository: Any a container or place where bowel excrement may be collected, for example, diaper, bedpan, colostomy bag, or toilet.

Unless otherwise explained, all technical and scientific terms used herein have the same meaning as commonly understood by one of ordinary skill in the art to which this invention belongs. The singular terms "a," "an," and "the" include plural referents unless context clearly indicates otherwise. Similarly, the word "or" is intended to include "and" unless the context clearly indicates otherwise. "Comprising" means "including." Hence "comprising A or B" means "including A or B," or "including A and B." Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including explanations of terms, will control. In addition, the materials, methods, and examples are illustrative only and not intended to be limiting.

### IV. Therapeutic Indications and Diagnosis

This disclosure provides methods and compositions for the treatment of chronic intestinal pseudo-obstruction. The subject to be treated is typically a mammal and preferably a human. Diagnosis of chronic intestinal pseudo-obstruction is well within the knowledge of those with skill in the art. Certain clinical features of chronic intestinal pseudo-obstruction and suggested diagnostic measures are described herein; however, one of skill in the art will recognize that other clinical features and diagnostic tests may be useful to identify subjects with chronic intestinal pseudo-obstruction.

The clinical features of chronic intestinal pseudo-obstruction typically include nausea, vomiting, early satiety, abdominal discomfort, distention, bloating, and anorexia, which is persistent and recurring. If stasis and vomiting are significant problems, there may be considerable weight loss, and disturbances of mineral and vitamin stores may result.

One or more of the symptoms of chronic intestinal pseudo-obstruction persist for at least one month, at least two months, at least three months, at least six months, at least one year, at least 5 years, or for a subject's lifetime. Though one of more of the symptoms of chronic intestinal pseudo obstruction may be acutely relieved by a single treatment, at least one symptom characteristic of the condition recurs at least one time and usually many times throughout the subject's lifetime. Following a single treatment to relieve at least one symptom of chronic intestinal pseudo-obstruction, at least one symptom characteristic of the condition recurs, for example, within at least one day of treatment, within at least two days of treatment, within at least three days of treatment, within at least five days of treatment, within at least one week of treatment, within at least two weeks of treatment, or within at least one month of treatment.

In the diagnosis of chronic intestinal pseudo-obstruction, it is usual to rule out the presence of mechanical obstruction of the bowel. Endoscopy (or more specifically, colonoscopy) and/or a small bowel x-ray are representative methods that may be used to exclude mechanical obstruction as a cause of the clinically observed symptoms. A CAT scan of the abdomen may also be useful to determine whether a mechanical bowel obstruction is present.

Diagnosis of chronic intestinal pseudo-obstruction may, but need not, be confirmed by functional tests. For example, a transit profile of the stomach or small bowel, or both, may be performed. These tests may be particularly helpful when scanning is done immediately after ingestion of a radiolabeled meal and then 2, 4, and 6 hours later.

Upper gastrointestinal manometry, using a multilumen tube with sensors strategically placed in the distal stomach and proximal small intestine, may be helpful to differentiate between a neuropathic and myopathic processes resulting in chronic intestinal pseudo-obstruction. Neuropathic conditions are characterized by normal contraction amplitude but uncoordinated contractile activity at the level of the stomach and small intestine. The neuropathic pressure profile shows a reduction in frequency of contractions, with normal contraction amplitude at the level of the stomach (antral hypomotility) or abnormalities in the propagation or coordination of fasting migrating motor complexes or postprandial motor patterns. Myopathic conditions are characterized by markedly reduced contraction amplitude but well-coordinated contractile activity.

In the presence of a neuropathic pattern of motor activity in the small intestine, it may be useful to pursue autonomic function tests. Autonomic testing may identify the presence of sympathetic adrenergic, sympathetic cholinergic, or vagal neuropathies. These abnormalities are revealed by orthostatic hypotension, changes in plasma norepinephrine levels when the patient is in the supine and standing positions, abnormal quantitative sudomotor axon reflex tests, and abnormal tests for vagal function (e.g., the heart interval change during deep breathing and the plasma pancreatic polypeptide response to modified sham feeding).

Chronic intestinal pseudo-obstruction is a clinical condition thought to have multiple etiologies. The particular cause of the condition is not critical to the practice of the methods and use of the compositions described herein, as long as on-going bowel evacuation in the subject may be achieved as described. Chronic intestinal pseudo-obstruction may arise, for example, as a result of neuropathic processes involving enteric and extrinsic nerves, and myopathic processes involving smooth muscle.

Processes involving the extrinsic nervous system include diabetic autonomic neuropathy, amyloidosis, and a paraneoplastic syndrome usually associated with small cell carcinoma of the lung. Surgical vagotomy also disrupts these nerves. Use of certain medications, such as .alpha..sub.2-adrenergic agonists, calcium channel blockers, anti-cholinergic drugs, or opiate agents (e.g., tricyclic antidepressants, nifedipine, narcotic analgesics, and antihypertensives such as clonidine), may lead to chronic intestinal pseudo-obstruction.

Disorders of the enteric nervous system that can result in chronic intestinal pseudo-obstruction may be the result of a degenerative, immune, or inflammatory process. Although the cause can only rarely be ascertained, chronic intestinal pseudo-obstruction may be induced by Norwalk virus, cytomegalovirus, and Epstein-Barr virus. Degenerative disorders associated with infiltration of the myenteric plexus with inflammatory cells, including eosinophils, have also been identified. Idiopathic chronic intestinal pseudo-obstruction is thought to occur in patients in whom there is no disturbance of extrinsic neural control and no underlying cause for the enteric neural abnormality.

Disturbances of smooth muscle, including progressive systemic sclerosis and amyloidosis, may result in chronic intestinal psuedo-obstruction. Sometimes dermatomyositis, dystrophia myotonica, and metabolic muscle disorders such as mitochondrial myopathy may be causes.

The methods and compositions described herein are believed to be useful for treating chronic intestinal pseudo-obstruction arising from or in association with the following non-exclusive list of conditions: chronic constipation, obstipation, idiopathic abdominal distention, abdominal pain, abdominal cramps, irritable bowel syndrome, megacolon associated with hypothyroidism, hypomotility of the colon associated with diabetes mellitus, neurological disorders, myopathic disorders, geriatric hypomotility disorders, jejunal-ileal bypass with secondary megacolon, hypomotility associated with cancer chemotherapy, hypomotility associated with severe burns and other major stresses, hypomotility associated with syndromes of depression, Parkinson's disease and other neurodegenerative disorders, post-operative intestinal distension, spinal cord injury, amyotrophic lateral sclerosis, spina bifida, multiple sclerosis, dementia and other pathological conditions.

### V. Methods of Use

The method disclosed herein comprises iontophoresis administration of a therapeutically effective amount of a drug combination comprising an acetylcholinesterase inhibitor and an anti-cholinergic agent to a subject having chronic intestinal pseudo-obstruction. For example, an iontophoresis device applies a positive current of approximately 4.0 mA per minute for 10 minutes across the skin of a subject in the presence of one or more iontophoresis patches impregnated with the drug agents in a 5:1 ratio by weight. In one embodiment, the one or more iontophoresis patches are impregnated with up to 15 mg of neostigmine and up to 3 mg of glycopyrrolate. In an alternative embodiment, the one or more iontophoresis patches are impregnated with up to 10 mg of neostigmine and up to 2 mg of glycopyrrolate. Table 1 below illustrates a comparison of iontophoresis administration at low and high doses versus an intravenous administration of the drug combination.

**Table 1**

| **Data** | **Intravenous (IV) Administration** | **Transdermal Administration-Low Dose** | **Transdermal Administration-High Dose** |
|---|---|---|---|
| Current Doses | NEO: 0.03mg/kg | NEO:0.05mg/kg | NEO:0.07mg/kg |
| | GLY: 0.006mg/kg | GLY: 0.01mg/kg | GLY:0.014mg/kg |
| # of Subjects | N=21 | N=18 | N=13 |
| # of Bowel Evacuations | N=16 | N=5 | N=5 |
| Mean Bowel Evacuation Time | t=21minutes | t=36minutes | t= 45minutes |
| # of Biological Responders (bowel evacuations and non-bowel evacuations) | N=20 | N= 12 | N=11 |
| Common Symptoms | Facial Twitching, Dry Mouth, Abdominal Cramping and Distension | Facial Twitching, Dry Mouth, Abdominal Cramping and Distension | Facial Twitching, Dry Mouth, Abdominal Cramping, and Distension |

Although the disclosed methods can be used prophylactically in any subject in a demographic group at significant risk for chronic intestinal pseudo-obstruction, subjects preferably will be selected using more specific criteria, such as a definitive diagnosis of chronic intestinal pseudo-obstruction. For example, treatment can be initiated (and continued as described herein) in a subject having signs and symptoms of chronic intestinal pseudo-obstruction, such as persistent, recurring bowel distension, constipation and abdominal pain in the absence of a mechanical blockage. In particular examples, the clinical picture will suggest chronic intestinal pseudo-obstruction, as in the case of persons with spinal cord injury, neurodegenerative disorders or myopathies.

Acetylcholinesterase inhibitors are well known in the art. Any acetylcholinesterase inhibitor can cause smooth muscle contractions in the gut sufficient to induce a single bowel evacuation in a subject without substantial toxic side effects in the subject. Representative acetylcholinesterase inhibitors include neostigmine, physostigmine, ambenonium, pyridostigmine, edrophonium, demecarium, echothiophate, and pralidoxime. According to the invention, the acetylcholinesterase inhibitor is neostigmine.

Anti-cholinergic agents are similarly well known in the art. Any anti-cholinergic agent is capable of reducing the cardiac side effects of an acetylcholinesterase inhibitor in a subject without having a substantial effect on the effect of the acetylcholinesterase inhibitor on gut motility in the subject. In addition, the anti-cholinergic agent preferably will not have substantial toxic side effects in the subject. Representative anti-cholinergic agents include glycopyrrolate, atropine, methscopolamine, homatropine, methantheline, propantheline, anisotropine, clidinium, hexocyclium, isopropamide, mepenzolate, oxyphenonium, or tridihexethyl. According to the invention, the anti-cholinergic agent is glycopyrrolate.

The disclosed methods contemplate a combination of an acetylcholinesterase inhibitor and an anti-cholinergic agent (individually, a drug, and collectively, drugs). A combination of the drugs envisions a physical combination of the drugs and/or a functional combination of the drugs.

A physical combination of an acetylcholinesterase inhibitor and an anti-cholinergic agent envisions the drugs combined in the same dosage form such as an pre-impregnated iontophoresis patch. For example, some methods contemplate that the drugs will be combined, in the therapeutically effective dosages, in a single composition that is pre-impregnated into an iontophoresis patch and amendable to administration by iontophoresis.

The methods described herein also contemplated a functional combination of an acetylcholinesterase inhibitor and an anti-cholinergic agent, which does not necessarily entail a physical combination of the drugs. In these embodiments, the drugs will have separate dosage forms, but will be administered so as to have substantially the same therapeutic effect as if administered in the same dosage form. For example, the drugs may be contained in different iontophoresis patches but be administered to the subject using iontophoresis at approximately the same time (that is, co-administration). Alternatively, the anti-cholinergic agent may be administered anytime after the acetylcholinesterase inhibitor as long as the anti-cholinergic agent is administered in a time frame that is sufficient to counter the cardiac side effects of the acetylcholinesterase inhibitor. For example, the anti-cholinergic agent may be administered between about 1 minute and about 20 minutes, or about 1 minute and about 10 minutes after the acetylcholinesterase inhibitor. More specifically, the anti-cholinergic agent may be administered no more than 1 minute, no more than 2 minutes, no more than 5 minutes, no more than 7 minutes, no more than 10 minutes, no more than 12 minutes, no more than 15 minutes, or no more than 20 minutes after the acetylcholinesterase inhibitor.

The disclosed drug combinations may be administered by any iontophoresis method that provides for delivery of therapeutically effective amounts of the drugs, for example, approximately milligram amounts (e.g., 0.1 mg to 5 mg) of the acetylcholinesterase inhibitor and approximately milligram amounts (e.g., 0.1 mg to 2.5 mg) of the anti-cholinergic agent in a time frame of up to one minute to within several minutes, for example within about 0.5 minute, within about 1 minute, within about 2 minutes, within about 3 minutes, within about 5 minutes, within about 7 minutes, within about 10 minutes or more.

In some embodiments, the invention provides for routine bowel care for a subject. The frequency of bowel care for each subject will preferably be determined by the subject, the subject's health care providers, or by the subject in consultation with health care providers. For example, routine bowel care may occur at least once each two weeks, at least once every ten days, at least once a week, at least twice a week, at least three times per week, at least four times per week, at least five times per week, at least six times per week, at least daily, or at least twice daily. The duration of treatment for chronic intestinal pseudo-obstruction, such as chronic pseudo-obstruction secondary to spinal cord injury, may be commensurate with the duration of the chronic condition, and in some examples is at least two weeks, at least one month, at least six months, at least one year or for a subject's lifetime.

Therapeutically effective doses of the disclosed drug combinations can be determined by one of skill in the art, with a goal of achieving a bowel evacuation event without substantial bradycardia (for example, symptomatic bradycardia) in a subject, for example, on a scheduled basis compatible with routine bowel care for the subject. Usual dosage ranges for clinically available acetylcholine esterase inhibitors and anti-cholinergic agents may be obtained from the Physicians' Desk Reference, 56th Edition (Mountvale, N.J.: Medical Economics Company, Inc., 2002).

An example of a dosage range for the acetylcholinesterase inhibitor, neostigmine, given by injection intravenously, intramuscularly or subcutaneously is, for each mode of administration, up to about 5 mg, up to about 4 mg, or up to about 2 mg in single or divided doses. Other representative dosage ranges for intravenous, intramuscular or subcutaneous injection of neostigmine include, for example, in the range of between about 0.1 mg to about 5 mg IV, between about 0.2 mg to about 4 mg, between about 0.3 mg and about 3 mg, between about 0.5 mg to about 2.0 mg in single or divided doses. In particular examples, dosages of neostigmine include, for example, 2 mg or 4 mg administered by either intravenous or intramuscular injection. In some subjects, for example in children, it may be advantageous to administer the acetylcholinesterase inhibitor on a per kilogram body weight basis; for example, in the range of between about 1.4 µg/kg to about 71 µg/kg, or between about 2.8 µg/kg to about 57 µg/kg, or between about 4.3 µg/kg to about 43 µg/kg, or between about 7.1 µg/kg to about 29 µg/kg administered intravenously or intramuscularly in single or divided doses.

An example of a dosage range for the anti-cholinergic agent, glycopyrrolate, administered intravenously or intramuscularly is, for each mode of administration, up to about 2.5 mg, up to about 1 mg, up to about 0.5 mg, up to about 0.4 mg, or up to about 0.2 mg in single or divided doses. In some examples, glycopyrrolate may be administered by intramuscular injection in the range of 0.001 mg to 0.01 mg/lb body weight in single or divided doses or, for example, at the dosage of 0.002 mg/lb body weight. Other representative dosage ranges for intravenous or intramuscular injection of glycopyrrolate include, for example, in the range of between about 0.1 mg to about 2.5 mg, between about 0.1 mg to about 1 mg, between about 0.1 mg and about 0.5 mg, between about 0.2 mg to about 0.4 mg in single or divided doses. In particular examples, dosages of glycopyrrolate include, for example, 0.2 mg or 0.4 mg administered by either intravenous or intramuscular injection. In some subjects, for example in children, it may be advantageous to administer the anti-cholinergic agent on a per kilogram body weight basis; for example, in the range of between about 1.4 µg/kg to about 38 µg/kg, or between about 1.4 µg/kg to about 15 µg/kg, or between about 1.4 µg/kg to about 7.1 µg/kg, or between about 2.8 µg/kg to about 5.7 µg/kg administered intravenously or intramuscularly in single or divided doses.

In some examples, the acetylcholinesterase inhibitor and anti-cholinergic agent are administered in a particular ratio of acetylcholinesterase inhibitor to anti-cholinergic agent by weight; for example, a ratio of about 2.5:1 to about 10:1, of about 2.5:1 to about 7:1, of about 3:1 to about 5:1. More particularly, in some examples the ratio of acetylcholinesterase inhibitor to anti-cholinergic agent by weight is 5:1 (e.g., 2 mg: 0.4 mg).

As discussed above, the disclosed methods envision that an acetylcholinesterase inhibitor and an anti-cholinergic agent may be (i) co-administered; or (ii) administered at approximately the same time by separate modes of administration; or (iii) administered at different times with administration of an acetylcholinesterase inhibitor preceding that of an anti-cholinergic agent.

The specific dose level and frequency of dosage for any particular subject may be varied and will depend upon a variety of factors, including the activity of the specific acetylcholinesterase inhibitor and/or anti-cholinergic agent, the metabolic stability and length of action of those compounds, the age, body weight, general health, sex, diet, mode and time of administration, rate of excretion, drug combination, and severity of the condition of the host undergoing therapy.

The disclosed methods may, but need not, be used in combination with traditional bowel care methods, such as laxatives, enemas and digital stimulation of the rectum. In some examples, digital stimulation of the rectum will be applied concurrent with administration of the disclosed drug combinations or slightly thereafter, for example within about 5 minutes of drug administration, or within about 10 minutes of drug administration, or within about 15 minutes of drug administration, or within about 20 minutes of drug administration, or within about 30 minutes of drug administration. Digital rectal stimulation in combination with the disclosed drug therapy may be particularly useful in patients whose anal sphincters contract in response to neostigmine administration; as may be the case, for example, with some persons with relatively recent spinal cord injuries.

### VI. Pharmaceutical Compositions

The disclosed drug combinations are administered to a subject in the form of a pharmaceutical composition. Pharmaceutical compositions comprising the disclosed drug combinations may be manufactured by means of conventional mixing, dissolving, granulating, levigating, emulsifying, encapsulating, entrapping or lyophilizing processes.

Pharmaceutical compositions may be formulated in conventional manner using one or more physiologically acceptable carriers, diluents, excipients or auxiliaries (such as wetting or emulsifying agents, preservatives, pH buffering agents and the like, for example, sodium acetate or sorbitan monolaurate), which facilitate processing of the active ingredients into preparations that can be used pharmaceutically. Examples of compositions and formulations suitable for pharmaceutical delivery of the agents herein disclosed are described in Remington's Pharmaceutical Sciences, by E. W. Martin, Mack Publishing Co., Easton, Pa., 15th Edition, 1975.

Systemic formulations include those designed for administration by iontophoresis. For iontophoresis, the compounds of the invention may be formulated in aqueous solutions, preferably in physiologically compatible buffers such as Hanks's solution, Ringer's solution, or physiological saline buffer, and then impregnated into an iontophoresis patch. The solution may contain formulatory agents such as suspending, stabilizing and/or dispersing agents. Alternatively, the compounds may be in powder form for constitution with a suitable vehicle, for example, sterile pyrogen-free water, before impregnation onto the iontophoresis patch.

### VII. Kits

The drug combinations disclosed herein can be supplied in the form of kits for use in prevention and/or other treatment of a disorder, condition or diseases (e.g., chronic intestinal pseudo-obstruction). In such a kit, a clinically effective amount of a combination of one or more acetylcholinesterase inhibitor(s) and one or more anti-cholinergic agent(s) is provided in one or more iontophoresis patches. In certain embodiments, the drug combination will be provided in the form of a single pharmaceutical composition.

In one embodiment, self administration kits may be provided for home bowel care. In one embodiment, an example kit for at home bowel care comprises a Dynatron iBox^{™} with high performance iontophoretic electrodes and a pre-filled medication. The Dynatron iBox^{™} delivers the electrical current necessary to drive the medication across the skin barrier and into the microvascular blood vessels. Typically only a single Dynatron iBox^{™} is needed for administering the drug combination. However, depending on the patient's weight and size, a second unit may be desirable in order to deliver a higher dose of the medications in a desired time frame. In one embodiment, the iontophoresis electrodes include a pre-impregnated patch and a single return electrode, which completes the electrical circuit. In one embodiment, each drug agent is separately impregnated onto a separate iontophoresis patch (e.g., a first patch for neostigmine and a second patch for glycopyrrolate). In accordance with the current commercially available concentration of the drug agents, if the desired volume of medication is greater than the capacity of the electrode patch (currently 4 ml), a second electrode with a second patch can be simultaneously employed. In an alternative embodiment, both drug agents can be pre-impregnated onto a single iontophoresis patch for more simple administration of both agents simultaneously from a single electrode patch. In such an embodiment, the drug agents are impregnated onto the single iontophoresis patch at a higher concentration.

Kits can also include instructions, usually written instructions, to assist the user in treating a disorder, condition or disease (e.g., treatment of chronic intestinal pseudo-obstruction) with the combination of one or more acetylcholinesterase inhibitor(s) and one or more anti-cholinergic agent(s). The instructions can be for use of the drug combination for any of the purposes described herein. Such instructions can optionally be provided on a computer readable medium.

The container(s) in which the drug combination is supplied can be any conventional container that is capable of holding the supplied form, for instance, iontophoresis patches. In some applications, the therapeutic drug combination may be provided in pre-measured single use amounts in individual, typically disposable, iontophoresis patches.

The amount of a drug combination supplied in the kit can be any appropriate amount, depending for instance on the market to which the product is directed. For instance, if the kit is adapted for clinical or home use, there may be several single-use iontophoresis patches containing the drug combination which may be used to provide for several treatments.

The following examples are provided to illustrate certain particular features and/or embodiments. These examples should not be construed to limit the invention to the particular features or embodiments described.

### EXAMPLES

### Example 1

### Decreased Colonic Motility Following Spinal Cord Injury

This example describes the effect of food and sleep on colonic motility in persons with spinal cord injury (SCI) as compared to the spinally intact (SI). This example demonstrates that colonic motility is decreased in SCI subjects; thus, such subjects may benefit from a therapeutic approach that increases bowel contractility.

### Methods

Eight (8) subjects with SCI (mean age of 59 years, mean duration of injury of 17 years, 5 paraplegics and 3 quadriplegics), and 6 SI subjects (mean age of 57 years) were investigated. Each SCI subject complained of difficulty with evacuation (DWE) at least once in the 6 months preceding testing.

Colonoscopy was performed after routine bowel preparation and all subjects had normal examinations. After colonoscopy, the proximal end of a solid-state pressure transducer catheter (separated by 10 cm) was tethered to the splenic flexure using endoclips (Olympus). The subjects were then allowed to carry out their usual daily activities for at least 24 hours. Data from the catheters were recorded on Gaeltec (Medical Measurements Inc.) portable recorders. The collected data were uploaded to a computer for analysis.

Motility index (as determined by software provided with the Gaeltec portable recorder system) was analyzed 1 hour before breakfast, during breakfast, 1 hour after breakfast, 1 hour prior to sleep, at sleep onset, and 1 hour after sleep onset. The motility index after an average 1 hour of sleep was used as the baseline value for each subject. The Student's t-test was used to determine significant difference.

### Results

The motility index determined at 1 hour pre-breakfast (3.6 vs. 9.3, p<0.01), during breakfast (4.6 vs. 14.3, p<0.02), and 1 hour post-breakfast (3.9 vs. 10.2, p<0.01) was significantly less for the SCI group as compared to the SI group. Post prandial colonic response was significant in both groups; however in the SCI group, this response was only evident in the proximal and not the distal leads. Likewise, the motility index determined at 1 hour pre-sleep onset (3.5 vs. 8.7, p<0.0005), at sleep onset (1.4 vs. 8.8, p<0.005), and 1 hour post-sleep onset (4.5 vs. 17.8, p<0.001) was significantly less for the SCI group as compared to the SI group. Sleep-induced depression in motility index was more significant in the SCI group (p<0.008). Both groups exhibited significant increases in the motility index during arousal.

This example demonstrates that DWE in SCI subjects is correlated with decreased colonic motility. The example further demonstrates that DWE may be due to phasic postprandial colonic response and a more exaggerated sleep-induced depression of colonic motility in individuals with SCI.

### Example 2 (comparative)

### Use of Neostigmine and Glycopyrrolate Combination Therapy to Stimulate Bowel Evacuation in Six Subjects with Spinal Cord Injury

This example describes the use of a combination of neostigmine and glycopyrrolate to stimulate bowel evacuation in SCI subjects without concomitant bradycardia.

Six (6) subjects with spinal cord injury (both paraplegic and quadriplegic) were tested. Bowel care times were typically 1-2 hours 3 times per week in these individuals. After bowel preparation and establishment of IV access, subjects were transferred to a fluoroscopic imaging table and, while in the supine position, underwent flexible sigmoidoscopy for placement of a solid state motility catheter in the descending colon. The motility apparatus was attached to the colon using a recently described technique that prevents migration of the sensors during the study (Fajardo et al., Gastrointest. Endosc., 51:199-201, 2000). Both the fluoroscopic image and the external end of the catheter were inputted to a system (Kay Elemetrics) that allowed simultaneous real time acquisition of fluoroscopic images and manometric data.

Under fluoroscopic control, the left side of the colon (rectum to splenic flexure) was filled with an oatmeal-barium paste using a bladder syringe and baseline manometric recordings were obtained for 5 minutes. Following these baseline measurements, each subject in random order was given either placebo or a combination of neostigmine (1 mg administered intravenously) and glycopyrrolate (0.2 mg administered intramuscularly). After each administration, manometric recordings were collected for an additional 30 minutes. Total fluoroscopic exposure time was limited to 10 minutes.

At the end of each measurement period, the residual oatmeal-barium paste was aspirated to determine residual "fecal" volume. If a subject failed to have a complete response to 1 mg of neostigmine and 0.2 mg glycopyrrolate, the subject was treated on a different day using a higher dose of neostigmine in the drug combination (i.e., 2 mg neostigmine and 0.4 mg glycopyrrolate administered intramuscularly). The vital signs (i.e., oxygen saturation, pulse and blood pressure) and respiratory status (i.e., auscultation) of each subject were continuously monitored.

Within 10 minutes of administration of the neostigmine-glycopyrrolate mixture, all subjects exhibited high amplitude colonic contractions. In addition, 5 out of 6 of the subjects demonstrated complete evacuation of the barium-oatmeal paste within 25 minutes, most within 15 minutes. No substantial bradycardia was observed in any of the subjects (for example, heart rate did not decrease below 45 BPM in any subject and/or none of the subjects experienced hypotension). The only side effect noted was transient muscle twitching in 2 of the subjects and minimal abdominal cramps in 1 of the subjects.

This comparative example demonstrates that combination therapy with neostigmine and glycopyrrolate can safely stimulate evacuation in a high proportion of individuals with spinal cord injury.

### Example 3 (comparative)

### Comparison of Bowel Evacuation Response in Thirteen Subjects with Spinal Cord Injury Treated with Neostigmine Alone or a Combination of Neostigmine and Glycopyrrolate

This example describes the effect of neostigmine alone and a combination of neostigmine and glycopyrrolate on bowel evacuation in the same subjects using the monitoring methods described in Example 2.

Thirteen (13) persons with SCI (5 quadriplegics, 8 paraplegics) with a mean age of 46 years (range 25-69) and mean duration of injury of 16 years (range 1-31) were treated. None of the subjects had known heart disease and/or history of arrhythmias.

On separate days, subjects were given by IV bolus either (i) 2 mg neostigmine alone, (ii) a combination of 2 mg neostigmine and 0.4 mg glycopyrrolate, or (iii) normal saline. Subjects were blinded to the type of infusion being delivered. The effect of these infusions on bowel evacuation was assessed semi-quantitatively on a scale of 0 (no evacuation) to 4+ (complete evacuation) using video fluoroscopy after rectal instillation of a barium-oatmeal paste having a consistency similar to that of stool. Blood pressure, heart rate and other vital signs were monitored in each subject. In addition, airway resistances were determined in each subject using the forced oscillation technique (as described, for example, in Goldman, Pulm. Pharmacol. Ther., 14(5):341-350, 2001). Total airway resistance was determined using 5 Hz forced oscillations, and central airway resistance was determined using 20 Hz forced oscillations.

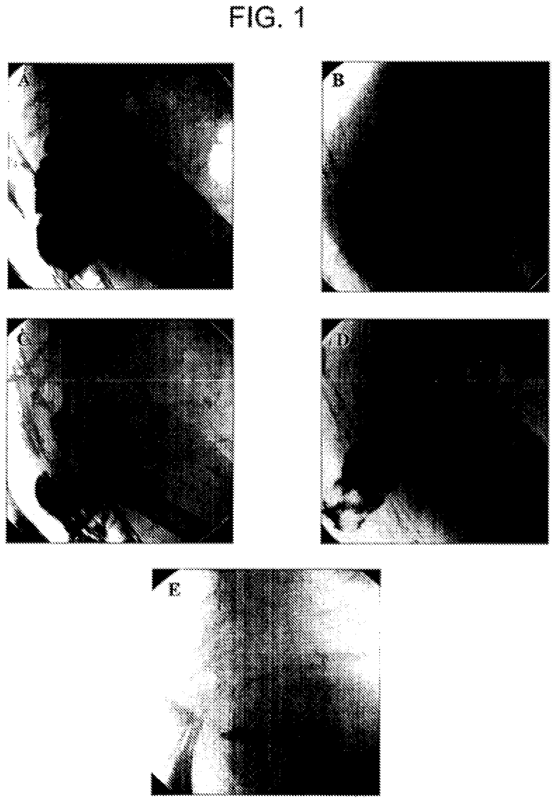

FIG. 1 shows examples of the bowel evacuation scoring method. FIG. 1A shows the fluoroscopy image from a subject having no response to the drug combination. This response received a 0 (zero) bowel evacuation score. FIGS. 1B-1E show progressively increasing bowel evacuation responses in different subjects. The responses in FIGS. 1B-1E correspond to bowel evacuation scores of 1+, 2+, 3+, and 4+, respectively.

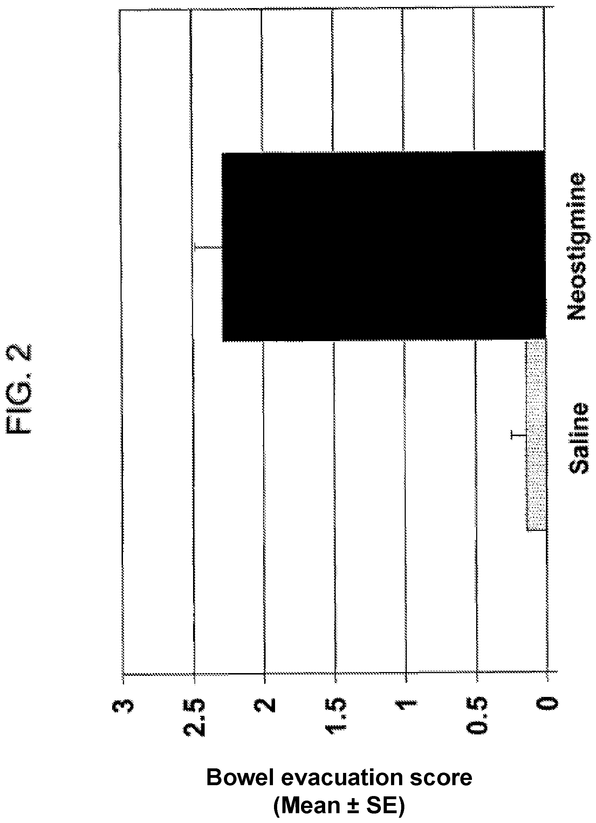

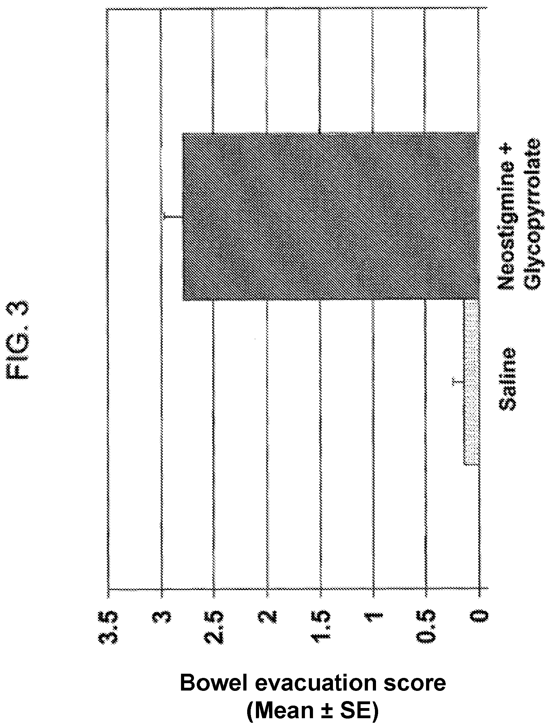

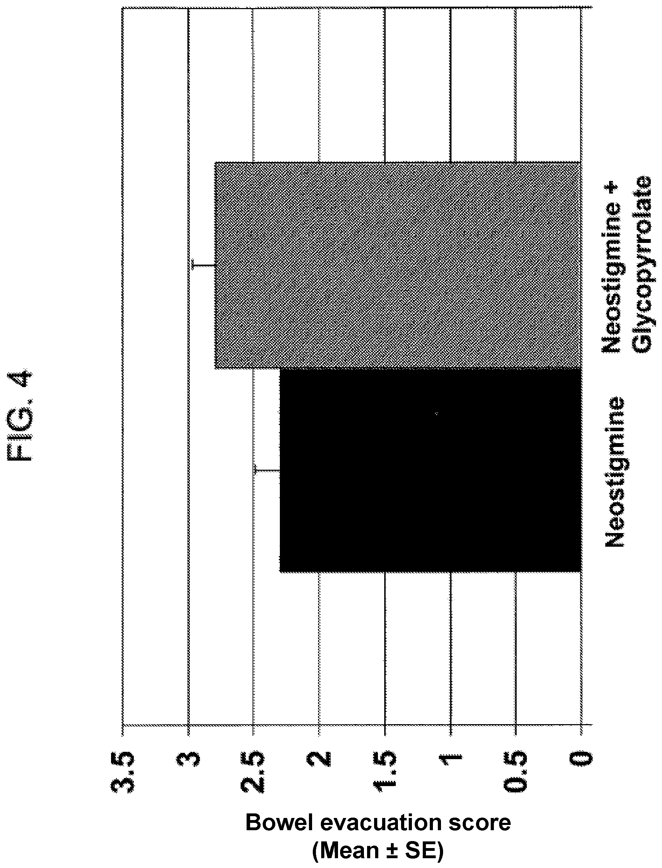

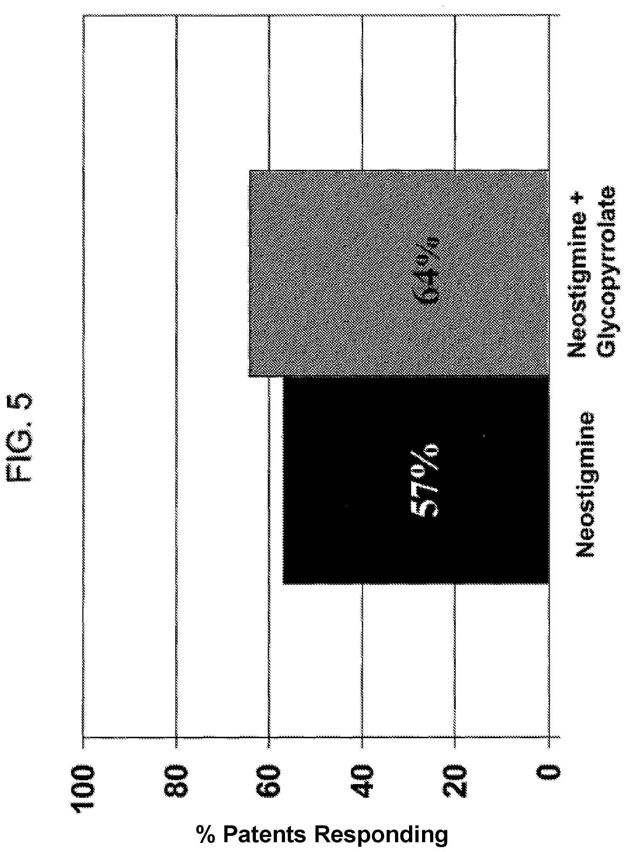

As shown in FIGS. 2 and 3, mean bowel evacuation score after neostigmine alone was about 2.29 (see FIG. 2), while mean evacuation score after neostigmine and glycopyrrolate was about 2.79 (see FIG. 3). No evacuation was observed over a 20 minute period after normal saline infusion (see FIGS. 2 and 3). Evacuation occurred in the range of 5-20 minutes (mean=11 minutes) post infusion of either neostigmine or the combination of neostigmine and glycopyrrolate. There was no difference between neostigmine or neostigmine and glycopyrrolate in terms of bowel evacuation score (see FIG. 4), the percentage of patients responding (see FIG. 5), or the rapidity of the bowel evacuation response.

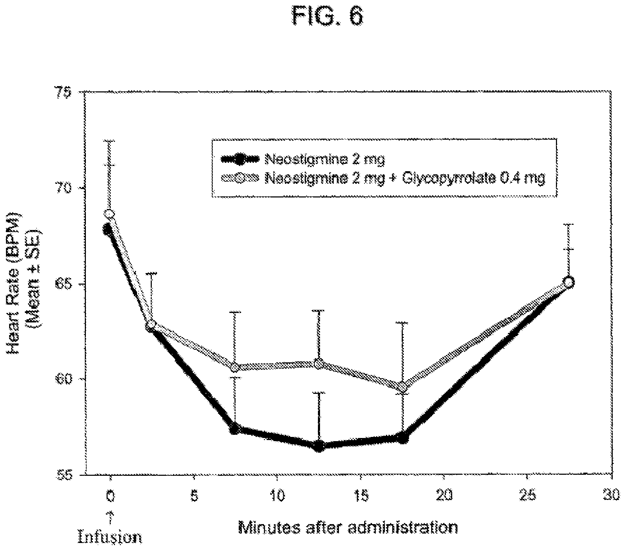

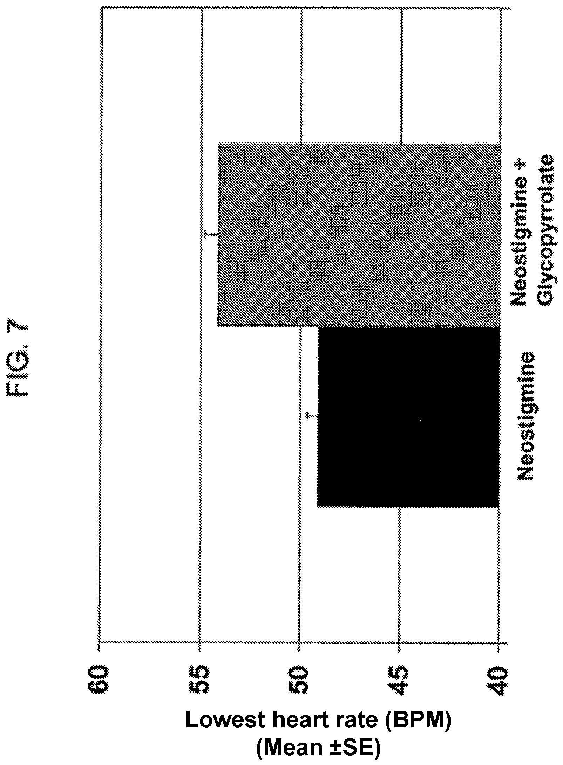

Quadriplegics and paraplegics did not differ in the likelihood of a response to neostigmine or neostigmine and glycopyrrolate. As shown in FIG. 6, subjects given the combination of neostigmine and glycopyrrolate experienced less bradycardia over a 30-minute time course than did subjects who received neostigmine alone. As shown in FIG. 7, the mean lowest heart rate measured in subjects given neostigmine alone (i.e., about 49 BMP) was significantly less (p=0.02) than that measured the subjects that received the combination of neostigmine and glycopyrrolate (i.e., about 54 BMP). No change in blood pressure was measured after administration of either neostigmine alone or the combination of neostigmine and glycopyrrolate. Other side effects, including muscle twitching, sweating, vague abdominal sensations and salivation, were transient and minimal.

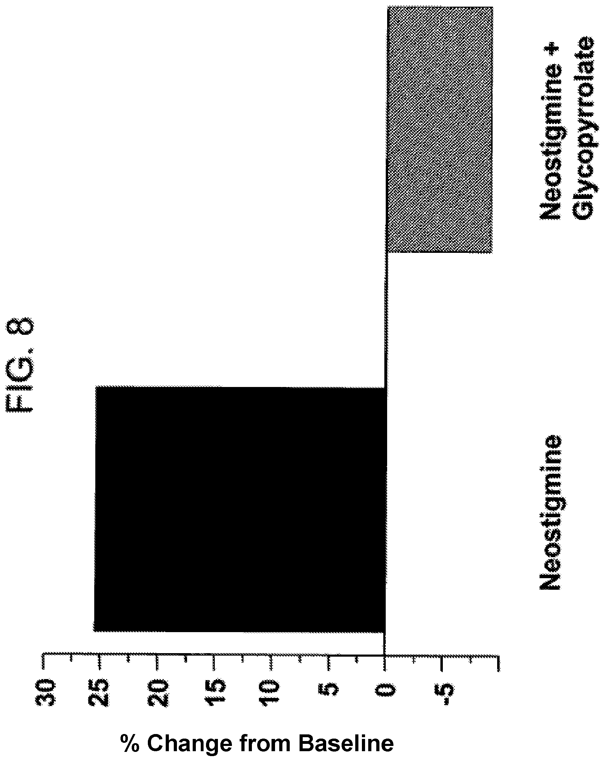

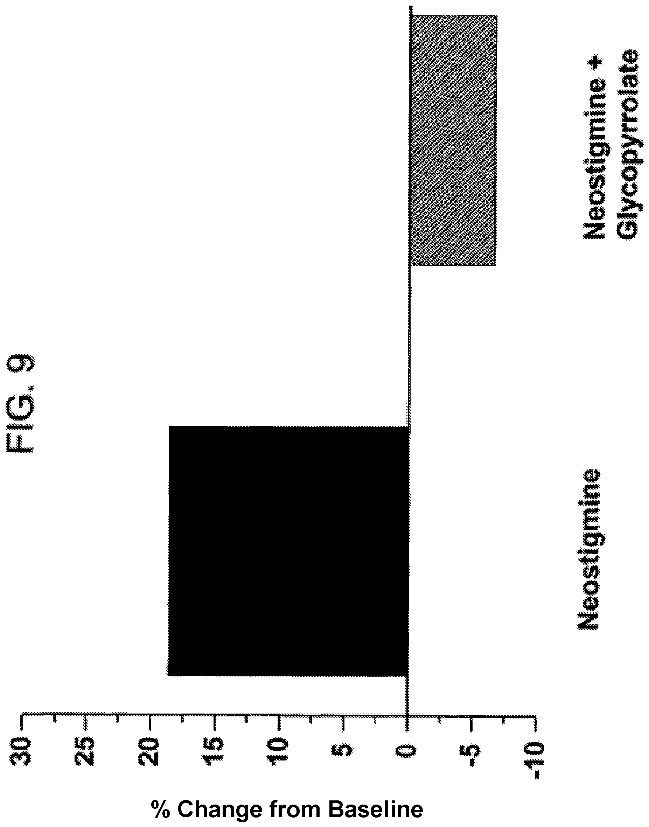

As shown in FIG. 8, total airway resistance increased approximately 25.5% over baseline upon administration of neostigmine alone. In comparison, total airway resistance decreased about 9% from baseline upon administration of the combination of neostigmine and glycopyrrolate. Similarly, FIG. 9 shows that central airway resistance increased approximately 18.4% over baseline upon administration of neostigmine alone. In comparison, central airway resistance decreased about 6.6% from baseline upon administration of the combination of neostigmine and glycopyrrolate. Taken together, these results indicate that administration of glycopyrrolate with neostigmine also counters respiratory side effects of neostigmine, such as bronchospasm. These results also indicate the usefulness of the drug combination for treatment of chronic intestinal pseudo-obstruction in patients with respiratory disorders, such as asthma.

This comparative example demonstrates that (i) the colonic response to neostigmine is not blunted by glycopyrrolate; (ii) neostigmine alone or the combination of neostigmine and glycopyrrolate produced prompt and complete evacuation in 57% and 64% of subjects, respectively, (iii) the combination of neostigmine and glycopyrrolate caused less bradycardia than neostigmine alone, and (iv) glycopyrrolate counteracts the respiratory side effects caused by neostigmine alone.

### Example 4 (comparative)

### Repeated Administration of Neostigmine and Glycopyrrolate in SCl Subjects

This example demonstrates the use of a combination of neostigmine and glycopyrrolate for routine, on-going bowel care for spinal cord injured patients (including both paraplegic and quadriplegic subjects).

Subjects' baseline responses to the combination of neostigmine and glycopyrrolate for bowel care were determined as described in Example 2. Thereafter, subjects received periodic (2-3 times per week) intramuscular injections of 1-2 mg neostigmine and 0.2-0.4 mg glycopyrrolate. The 1 mg dose of neostigmine (with 0.2 mg glycopyrrolate) was used for subjects who had a complete evacuation of the oatmeal-barium bolus during the initial baseline treatment and the 2 mg dose of neostigmine (with 0.4 mg glycopyrrolate) was used for all other subjects.

Following administration of the drug combination, the subjects underwent their usual bowel care routines until satisfactory bowel evacuation was achieved. The treatments were continued for at least a month and are on-going.

Chronic administration of neostigmine and glycopyrrolate as described in this comparative example decreased the bowel care time of the subjects by approximately 50-75% as compared to traditional methods of bowel care.

### Example 5 (comparative)

### Repeated Administration of Neostigmine and Glycopyrrolate in Rectal Suppository Formulation

### Formulation of Rectal Suppository

The drug combinations disclosed herein, such as a combination of neostigmine and glycopyrrolate, are formulated with a suppository vehicle adapted for rectal administration. Methods of formulating rectal suppositories are well known in the art.

A typical rectal suppository includes a suppository base, certain adjuvenants and additives suitable for making such formulations. The suppository base may be an aqueous or a fatty base material, the latter being preferred mainly for ease of formulation and administration. Fatty base materials useful in a suppository base include, for example fatty oils and fats (such as, cocoa butter, palm oil, coconut oil, or lard), waxes (such as, lanolin and vasoline), or fatty acids (such as, oleic, stearic, and lauric acids).

The suppository preparations may also contain other additives, such as antioxidants (such as, butylated hydroxyanisole (BHA) or butylated hydroxytoluene (BHT)), stabilizers, viscosity builders, preservatives, and the like. The concentration of these additives may vary according to the particular additive used and the desired result sought. The use, the kind, and the concentration of additives for suppository preparations are within the knowledge of the ordinarily skilled artisan. In particular examples, agents may be added to promote the absorption of the active ingredients contained in the suppository (such as, a combination of neostigmine and glycopyrrolate) by the intestinal or rectal mucosa.

A combination of neostigmine and glycopyrrolate may be incorporated into the suppository vehicle using any method(s) known in the art. For example, neostigmine and glycopyrrolate may be incorporated into a suppository vehicle by the use of a buffer system, a gelatin stabilizer, or a bulking agent.

In some comparative examples, a buffer system may be used as a carrier for a combination of neostigmine and glycopyrrolate. The buffer provides stability for the drugs and facilitates admixture with the components of the suppository vehicle. A representative buffer system may contain sodium or potassium phosphate/phosphoric acid buffer, or sodium or potassium acetate/acetic acid buffer, or sodium or potassium citrate/citric acid buffer. The concentration and pH of the buffer are preferably those values in which the drug combination is stable. For example, the concentration of the buffer may be in the range of 0.01 M to 0.5 M or in the range of 0.05 M to 0.2 M. A useful pH range for the buffer system is between 2 0 to 8.0.

In other comparative examples, a stabilizer system may be used as a carrier for the combination of neostigmine and glycopyrrolate. A representative stabilizer system may contain from about 1 to about 32% w/w of a gelatin hydrolized in a 0.9% w/w sodium chloride solution or in purified water. A useful pH range for a stabilizer system is between 2.0 to 8.0.

In yet other comparative examples, a lyophilized or dry mixed bulking agent may be used as a carrier for the combination of neostigmine and glycopyrrolate. Examples of bulking agents include, for example, gelatin, methionine, dextrose, sucrose, mannitol, sorbitol, lactose, methyl cellulose, povidone, sodium chloride and sodium acetate.

The components are the suppository may be combined, for example, by melting the suppository base with any absorption promoter, adding antioxidants and/or other additives, and adding a combination of neostigmine (e.g., 0.1-5.0 mg) and glycopyrrolate (e.g., 0.1-2.5 mg) suspended in a buffer solution or in a stabilizer system or homogenously distributed in a bulk powder mix. The mixture is blended and poured into suitable suppository molds and allowed to solidify.

### Use of Rectal Suppository for Chronic, On-going Bowel Care

Subjects' baseline responses to the combination of neostigmine and glycopyrrolate in a rectal suppository formulation are determined as described in Example 2. Thereafter, a subject inserts (or has inserted by a home-care health care provider) a suppository into his or her rectum 2-3 times per week. Following insertion of the suppository, the subject will undergo his or her usual bowel care routine until satisfactory bowel evacuation is achieved. The treatments are continued for at least two weeks, but may continue for months, years, or until otherwise decided by the subject and/or his or her health care team.

### Example 6 (comparative)

### Repeated Administration of Neostigmine and Glycopyrrolate by Indwelling Catheter

This comparative example describes the chronic administration of a combination of neostigmine and glycopyrrolate by intravenous administration using an indwelling catheter, in particular a Port-A-Cath.RTM. catheter.

An indwelling catheter is tube that is implanted for a prolonged period of time (such as, weeks, months or years) with one end of the tube located within the body (such as, within a large vein) and the other end of the tube remaining accessible from the outside of the body. An indwelling catheter facilitates long-term intravenous drug therapy. Several types of indwelling catheters are known in the art, for example, the Port-A-Cath.RTM., the Hickman catheter, and a PICC line (i.e., peripherally inserted central line). The Port-A-Cath.RTM. catheter is a well-known device having an injection port, which placed surgically under the skin of the arm or chest, and an attached catheter, which is inserted into a large vein in order to administer drugs to a subject. The Port-A-Cath.RTM. catheter is advantageous because it is less visible than the other types of indwelling catheters and it has less chance of infection because the injection port is located under the skin.

A subject with chronic intestinal pseudo-obstruction is identified as described herein; including, for example, spinal cord injured persons (such as paraplegic or quadriplegic persons). The subject's baseline responses to the combination of neostigmine and glycopyrrolate (in the amounts of 2 mg neostigmine plus 0.4 mg glycopyrrolate, or 1 mg neostigmine plus 0.2 mg glycopyrrolate) in an intravenous formulation are determined as described in Example 2.

Thereafter, the subject receives a subcutaneous port by implantation under general anesthesia. The surgeon makes a small incision in the chest where the subcutaneous portal is placed and another incision near the collarbone where the catheter enters a vein in the lower part of the neck. One end of the catheter is placed in the large blood vessel of the neck and threaded into the right atrium of the heart. The other end of the catheter is tunneled under the skin where it is attached to the portal. Fluid is injected into the portal to ensure that the device works properly. The portal is then placed under the skin in the right chest and stitched to the underlying muscle. Both incisions are then closed.

Once the implantation wounds have healed, the subject uses the indwelling catheter for intravenous injection of a combination of neostigmine and glycopyrrolate at the dosage determined to be appropriate in the initial examination. A topical anesthesia, such as EMLA cream or ethyl chloride, is applied to the skin above the portal prior to the needle poke. The subject or his or her health care provider inserts the needle of the syringe containing the drug combination into the subcutaneous injection port and administers the drug combination to the subject by injection. The injection port septum is self-sealing after needle removal, and is designed to withstand years of needle insertions.

Following administration of the drug combination, the subject undergoes his or her usual bowel care routine until satisfactory bowel evacuation is achieved. The treatments continue for as long as desired by the subject in consultation with his or her health care team.

### Example 7

Difficulty with evacuation is a common clinical problem after spinal cord injury (SCI) and other neurological or bowel disorders. Intravenous (IV) administration of neostigmine and glycopyrrolate (NEO/GLY) safely and rapidly promotes bowel evacuation, however, because IV administration of these agents is of limited practical value for routine bowel care. In this Example 7, an electric field was employed to non-invasively drive polar ions across the skin and correspondingly drive the medication across the skin barrier and into the microvascular blood vessels of the subject to achieve safe, predictable bowel evacuation. The goal of Example 7 was to determine whether transdermal (TD) administration of NEO/GLY by ION technology promotes increased bowel motility and evacuation with an acceptable safety profile.

Persons with SCI who had a past history of cardiovascular or respiratory complications were excluded from Example 7. The ION system consisted of Dynatron iBoxes^{™} that delivered a positive current of 4.0 mA-min via a dermal patch for 10 minutes. Referring back to Table 1, at the initial visit, subjects were screened for responsiveness to IV NEO (0.03 mg/kg)/GLY (0.006 mg/kg). Subjects who had a positive response to IV NEO/GLY then progressed at the second visit to receive low-dose TD NEO (0.05 mg/kg)/GLY (0.01 mg/kg). Those who failed to have drug-related signs and/or symptoms and/or a bowel movement after administration of low-dose TD NEO/GLYwere administered high-dose TD NEO (0.07 mg/kg)/GLY (0.014 mg/kg) at the third visit. After TD drug administration, defecation, increased bowel sounds, bradycardia, bronchoconstriction, and other drug-related cholinergic side effects were assessed every 5 min for 1 hour.

A total of twenty-three subjects were screened with IV NEO/GLY and seventeen of the twenty-three subjects had a bowel movement (mean: 20.7min) and twenty-two of twenty-three subjects reported drug-related side effects (i.e., presence of eye twitching, cramping, and/or defecation). Twenty subjects who reported a positive drug-related response after IV NEO/GLY progressed to the second visit. After low-dose TD NEO/GLY, five of twenty subjects (25%) had bowel evacuation (mean: 36 min), and twelve subjects (60%) had drug-related responses. Fourteen subjects who did not have cholinergic responses at the second visit progressed to receive a higher dose of TD NEO/GLY at the third visit. Five of fourteen subjects (35.7%) had bowel evacuation (mean 25.6 min) and twelve (85.7%) had drug-related responses. Overall, 50% of subjects had bowel evacuations with either low-dose or high-dose TD NEO/GLY. Blood pressure, heart rate, oxygen saturation, and pulmonary measurements were not clinically altered during any of the visits. No statistical differences were observed in bowel evacuation time between IV screening vs. either low dose (p=0.10) or high dose (p=0.68) TD NEO/GLY.

Accordingly, transdermal NEO/GLY can be safely administered by iontophoresis to induce increased bowel motility and evacuation. Thus, transdermal drug delivery of NEO/GLY by iontophoresis improves bowel care in persons with SCI and can also be used for persons with other conditions characterized by prolonged colonic transit time.

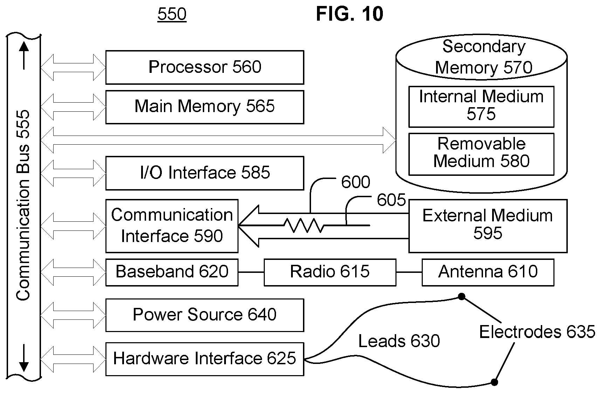

FIG. 10 is a block diagram illustrating an example processor enabled wired or wireless system 550 that may be used in connection with various embodiments described herein. For example the system 550 may be used as or in conjunction with an iontophoresis apparatus as previously discussed herein. In various embodiments, the system 550 can be implemented as part of a conventional personal computer, personal digital assistant, smart phone, tablet computer, or any other general purpose or specific purpose processor enabled device. For example, in one embodiment the system 550 may be implemented as a Dynatron ^{®} iBox^{™} iontophoresis device. Other computer systems and/or architectures may be also used, as will be clear to those skilled in the art.

The system 550 preferably includes one or more processors, such as processor 560. Additional processors may be provided, such as an auxiliary processor to manage input/output, an auxiliary processor to perform floating point mathematical operations, a special-purpose microprocessor having an architecture suitable for fast execution of signal processing algorithms (e.g., digital signal processor), a slave processor subordinate to the main processing system (e.g., back-end processor), an additional microprocessor or controller for dual or multiple processor systems, or a coprocessor. Such auxiliary processors may be discrete processors or may be integrated with the processor 560.

The processor 560 is preferably connected to a communication bus 555. The communication bus 555 may include a data channel for facilitating information transfer between storage and other peripheral components of the system 550. The communication bus 555 further may provide a set of signals used for communication with the processor 560, including a data bus, address bus, and control bus (not shown). The communication bus 555 may comprise any standard or non-standard bus architecture such as, for example, bus architectures compliant with industry standard architecture ("ISA"), extended industry standard architecture ("EISA"), Micro Channel Architecture ("MCA"), peripheral component interconnect ("PCI") local bus, or standards promulgated by the Institute of Electrical and Electronics Engineers ("IEEE") including IEEE 488 general-purpose interface bus ("GPIB"), IEEE 696/S-100, and the like.

System 550 preferably includes a main memory 565 and may also include a secondary memory 570. The main memory 565 provides storage of instructions and data for programs executing on the processor 560. The main memory 565 is typically semiconductor-based memory such as dynamic random access memory ("DRAM") and/or static random access memory ("SRAM"). Other semiconductor-based memory types include, for example, synchronous dynamic random access memory ("SDRAM"), Rambus dynamic random access memory ("RDRAM"), ferroelectric random access memory ("FRAM"), and the like, including read only memory ("ROM").

The secondary memory 570 may optionally include a internal memory 575 and/or a removable medium 580, for example a floppy disk drive, a magnetic tape drive, a compact disc ("CD") drive, a digital versatile disc ("DVD") drive, etc. The removable medium 580 is read from and/or written to in a well-known manner. Removable storage medium 580 may be, for example, a floppy disk, magnetic tape, CD, DVD, SD card, etc.

The removable storage medium 580 is a non-transitory computer readable medium having stored thereon computer executable code (i.e., software) and/or data. The computer software or data stored on the removable storage medium 580 is read into the system 550 for execution by the processor 560.

In alternative embodiments, secondary memory 570 may include other similar means for allowing computer programs or other data or instructions to be loaded into the system 550. Such means may include, for example, an external storage medium 595 and an interface 570. Examples of external storage medium 595 may include an external hard disk drive or an external optical drive, or and external magneto-optical drive.

Other examples of secondary memory 570 may include semiconductor-based memory such as programmable read-only memory ("PROM"), erasable programmable read-only memory ("EPROM"), electrically erasable read-only memory ("EEPROM"), or flash memory (block oriented memory similar to EEPROM). Also included are any other removable storage media 580 and communication interface 590, which allow software and data to be transferred from an external medium 595 to the system 550.

System 550 may also include an input/output ("I/O") interface 585. The I/O interface 585 facilitates input from and output to external devices. For example the I/O interface 585 may receive input from a keyboard or mouse and may provide output to a display. The I/O interface 585 is capable of facilitating input from and output to various alternative types of human interface and machine interface devices alike.

System 550 may also include a communication interface 590. The communication interface 590 allows software and data to be transferred between system 550 and external devices (e.g. printers), networks, or information sources. For example, computer software or executable code may be transferred to system 550 from a network server via communication interface 590. Examples of communication interface 590 include a modem, a network interface card ("NIC"), a wireless data card, a communications port, a PCMCIA slot and card, an infrared interface, and an IEEE 1394 fire-wire, just to name a few.

Communication interface 590 preferably implements industry promulgated protocol standards, such as Ethernet IEEE 802 standards, Fiber Channel, digital subscriber line ("DSL"), asynchronous digital subscriber line ("ADSL"), frame relay, asynchronous transfer mode ("ATM"), integrated digital services network ("ISDN"), personal communications services ("PCS"), transmission control protocol/Internet protocol ("TCP/IP"), serial line Internet protocol/point to point protocol ("SLIP/PPP"), and so on, but may also implement customized or non-standard interface protocols as well.

Software and data transferred via communication interface 590 are generally in the form of electrical communication signals 605. These signals 605 are preferably provided to communication interface 590 via a communication channel 600. In one embodiment, the communication channel 600 may be a wired or wireless network, or any variety of other communication links. Communication channel 600 carries signals 605 and can be implemented using a variety of wired or wireless communication means including wire or cable, fiber optics, conventional phone line, cellular phone link, wireless data communication link, radio frequency ("RF") link, or infrared link, just to name a few.

Computer executable code (i.e., computer programs or software) is stored in the main memory 565 and/or the secondary memory 570. Computer programs can also be received via communication interface 590 and stored in the main memory 565 and/or the secondary memory 570. Such computer programs, when executed, enable the system 550 to perform the various functions of the present invention as previously described.

In this description, the term "computer readable medium" is used to refer to any non-transitory computer readable storage media used to provide computer executable code (e.g., software and computer programs) to the system 550. Examples of these media include main memory 565, secondary memory 570 (including internal memory 575, removable medium 580, and external storage medium 595), and any peripheral device communicatively coupled with communication interface 590 (including a network information server or other network device). These non-transitory computer readable mediums are means for providing executable code, programming instructions, and software to the system 550.

In an embodiment that is implemented using software, the software may be stored on a computer readable medium and loaded into the system 550 by way of removable medium 580, I/O interface 585, or communication interface 590. In such an embodiment, the software is loaded into the system 550 in the form of electrical communication signals 605. The software, when executed by the processor 560, preferably causes the processor 560 to perform the inventive features and functions previously described herein.

The system 550 also includes optional wireless communication components that facilitate wireless communication over a voice and over a data network. The wireless communication components comprise an antenna system 610, a radio system 615 and a baseband system 620. In the system 550, radio frequency ("RF") signals are transmitted and received over the air by the antenna system 610 under the management of the radio system 615.

In one embodiment, the antenna system 610 may comprise one or more antennae and one or more multiplexors (not shown) that perform a switching function to provide the antenna system 610 with transmit and receive signal paths. In the receive path, received RF signals can be coupled from a multiplexor to a low noise amplifier (not shown) that amplifies the received RF signal and sends the amplified signal to the radio system 615.

In alternative embodiments, the radio system 615 may comprise one or more radios that are configured to communicate over various frequencies. In one embodiment, the radio system 615 may combine a demodulator (not shown) and modulator (not shown) in one integrated circuit ("IC"). The demodulator and modulator can also be separate components. In the incoming path, the demodulator strips away the RF carrier signal leaving a baseband receive audio signal, which is sent from the radio system 615 to the baseband system 620.

If the received signal contains audio information, then baseband system 620 decodes the signal and converts it to an analog signal. Then the signal is amplified and sent to a speaker. The baseband system 620 also receives analog audio signals from a microphone. These analog audio signals are converted to digital signals and encoded by the baseband system 620. The baseband system 620 also codes the digital signals for transmission and generates a baseband transmit audio signal that is routed to the modulator portion of the radio system 615. The modulator mixes the baseband transmit audio signal with an RF carrier signal generating an RF transmit signal that is routed to the antenna system and may pass through a power amplifier (not shown). The power amplifier amplifies the RF transmit signal and routes it to the antenna system 610 where the signal is switched to the antenna port for transmission.

The baseband system 620 is also communicatively coupled with the processor 560. The central processing unit 560 has access to data storage areas 565 and 570. The central processing unit 560 is preferably configured to execute instructions (i.e., computer programs or software) that can be stored in the memory 565 or the secondary memory 570. Computer programs can also be received from the baseband processor 610 and stored in the data storage area 565 or in secondary memory 570, or executed upon receipt. Such computer programs, when executed, enable the system 550 to perform the various functions of the present invention as previously described. For example, data storage areas 565 may include various software modules (not shown) that are executable by processor 560.

Hardware interface 625 is configured to provide an interface between one or more electrodes 635 that are electrically connected to the system 550 by one or more leads 630. In one embodiment, the processor 560 controls the delivery of a desired amount of electrical current from the power source 640 (e.g., batteries) via the leads 630 to the electrodes 635. In one embodiment, the system 550 is configured to deliver electrical current in the range of 0.5 mA to 8.0 mA.

Various embodiments may also be implemented primarily in hardware using, for example, components such as application specific integrated circuits ("ASICs"), or field programmable gate arrays ("FPGAs"). Implementation of a hardware state machine capable of performing the functions described herein will also be apparent to those skilled in the relevant art. Various embodiments may also be implemented using a combination of both hardware and software.

Furthermore, those of skill in the art will appreciate that the various illustrative logical blocks, modules, circuits, and method steps described in connection with the above described figures and the embodiments disclosed herein can often be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled persons can implement the described functionality in varying ways for each particular application, but such implementation decisions should not be interpreted as causing a departure from the scope of the invention. In addition, the grouping of functions within a module, block, circuit or step is for ease of description. Specific functions or steps can be moved from one module, block or circuit to another without departing from the invention.

Moreover, the various illustrative logical blocks, modules, and methods described in connection with the embodiments disclosed herein can be implemented or performed with a general purpose processor, a digital signal processor ("DSP"), an ASIC, FPGA or other programmable logic device, discrete gate or transistor logic, discrete hardware components, or any combination thereof designed to perform the functions described herein. A general-purpose processor can be a microprocessor, but in the alternative, the processor can be any processor, controller, microcontroller, or state machine. A processor can also be implemented as a combination of computing devices, for example, a combination of a DSP and a microprocessor, a plurality of microprocessors, one or more microprocessors in conjunction with a DSP core, or any other such configuration.

Additionally, the steps of a method or algorithm described in connection with the embodiments disclosed herein can be embodied directly in hardware, in a software module executed by a processor, or in a combination of the two. A software module can reside in RAM memory, flash memory, ROM memory, EPROM memory, EEPROM memory, registers, hard disk, a removable disk, a CD-ROM, or any other form of storage medium including a network storage medium. An exemplary storage medium can be coupled to the processor such the processor can read information from, and write information to, the storage medium. In the alternative, the storage medium can be integral to the processor. The processor and the storage medium can also reside in an ASIC.

## Claims

1. A pharmaceutical composition comprising a drug combination comprising neostigmine and glycopyrrolate for use in a method of bowel care, the method comprising administering by iontophoresis a therapeutically effective amount of the pharmaceutical composition to a subject having chronic intestinal pseudo-obstruction to relieve chronic constipation,
wherein the ratio of neostigmine to glycopyrrolate is 2.5:1 to 10:1 by weight, and
wherein the iontophoresis comprises
placing one or more patches, collectively impregnated with the therapeutically effective amount of the drug combination, on the subject's skin, wherein each of the one or more patches is paired with an electrical lead, and
delivering an electric current through the electrical lead for each of the one or more patches to drive the therapeutically effective amount of the drug combination across the subject's skin barrier into the subject's microvascular blood vessels.

2. The pharmaceutical composition for use according to claim 1, wherein the therapeutically effective amount of the drug combination comprises a ratio of neostigmine to glycopyrrolate of about 5:1 by weight.

3. The pharmaceutical composition for use according to claim 1, wherein the chronic intestinal pseudo-obstruction is a result of spinal cord injury, and wherein the spinal cord injury results in paraplegia or quadriplegia.

4. The pharmaceutical composition for use according to claim 1, wherein delivering the electric current through the electrical lead comprises delivering a positive current of about 4.0 mA per minute for about 10 minutes.

5. The pharmaceutical composition for use according to claim 1, wherein the one or more patches are impregnated with up to 15 mg of the neostigmine and up to 3 mg of the glycopyrrolate.

6. The pharmaceutical composition for use according to claim 1, wherein the one or more patches are impregnated with up to 10 mg of the neostigmine and up to 2 mg of the glycopyrrolate.

7. The pharmaceutical composition for use according to claim 1, wherein the one or more patches are impregnated with 0.07 mg of the neostigmine per kilogram of the subject's weight and 0.014 mg of the glycopyrrolate per kilogram of the subject's weight.

8. The pharmaceutical composition for use according to claim 1, wherein the one or more patches are impregnated with 0.05 mg of the neostigmine per kilogram of the subject's weight and 0.01 mg of the glycopyrrolate per kilogram of the subject's weight.

9. The pharmaceutical composition for use according to claim 1, wherein the iontophoresis delivers the therapeutically effective amount of the drug combination at 0.1 mg to 5 mg of the neostigmine per minute and 0.1 mg to 2.5 mg of the glycopyrrolate per minute.

10. The pharmaceutical composition for use according to claim 1, wherein the chronic intestinal pseudo-obstruction is a result of one or more of chronic constipation, idiopathic abdominal distension, abdominal pain, abdominal cramps, irritable bowel syndrome, megacolon associated with hyperthyroidism, hypomotility of the colon associated with diabetes mellitus, a neurological disorder, a myopathic disorder, geriatric hypomotility disorder, jejunal-ileal bypass with secondary megacolon, hypomotility associated with cancer chemotherapy, hypomotility associated with burns or stresses, hypomotility associated with depression, Parkinson's disease, post-operative intestinal distension, amyotrophic lateral sclerosis, spina bifida, multiple sclerosis, or dementia.

11. The pharmaceutical composition for use according to claim 1, wherein the administration occurs at least one time per week over a period of at least one month.

12. The pharmaceutical composition for use according to claim 11, wherein the administration occurs over a period of at least six months.

13. The pharmaceutical composition for use according to claim 1, wherein the administration occurs at least three times per week over a period of at least one month.

## Patentansprüche

1. Pharmazeutische Zusammensetzung umfassend eine Arzneimittelkombination umfassend Neostigmin und Glycopyrrolat zur Verwendung in einem Verfahren zur Darmpflege, wobei das Verfahren die Verabreichung einer therapeutisch wirksamen Menge der pharmazeutischen Zusammensetzung durch Iontophorese an einen Patienten mit chronischer intestinaler Pseudoobstruktion umfasst, um chronische Obstipation zu lindern,
wobei das Gewichtsverhältnis von Neostigmin zu Glycopyrrolat 2,5:1 bis 10:1 beträgt, und wobei die Iontophorese umfasst:
- Aufbringung eines oder mehrerer Pflaster, die durchgehend mit der therapeutisch wirksamen Menge der Arzneimittelkombination getränkt sind, auf die Haut des Patienten, wobei jedes der einen oder mehreren Pflaster mit einer elektrischen Leitung versehen ist, und
- Zuführung eines elektrischen Stroms durch die elektrische Leitung für jedes der einen oder mehreren Pflaster, um die therapeutisch wirksame Menge der Arzneimittelkombination durch die Hautbarriere des Patienten in die mikrovaskulären Blutgefäße des Patienten abzugeben.

2. Pharmazeutische Zusammensetzung zur Verwendung nach Anspruch 1, wobei die therapeutisch wirksame Menge der Arzneimittelkombination ein Gewichtsverhältnis von Neostigmin zu Glycopyrrolat von etwa 5:1 umfasst.

3. Pharmazeutische Zusammensetzung zur Verwendung gemäß Anspruch 1, wobei die chronische intestinale Pseudoobstruktion eine Folge einer Rückenmarksverletzung ist, und wobei die Rückenmarksverletzung zu einer Paraplegie oder Quadriplegie führt.

4. Pharmazeutische Zusammensetzung zur Verwendung gemäß Anspruch 1, wobei die Zuführung des elektrischen Stroms durch die elektrische Leitung die Zuführung eines positiven Stroms von etwa 4,0 mA pro Minute für etwa 10 Minuten umfasst.

5. Pharmazeutische Zusammensetzung zur Verwendung nach Anspruch 1, wobei das eine oder die mehreren Pflaster mit bis zu 15 mg Neostigmin und bis zu 3 mg Glycopyrrolat getränkt sind.

6. Pharmazeutische Zusammensetzung zur Verwendung nach Anspruch 1, wobei das eine oder die mehreren Pflaster mit bis zu 10 mg Neostigmin und bis zu 2 mg Glycopyrrolat getränkt sind.

7. Pharmazeutische Zusammensetzung zur Verwendung nach Anspruch 1, wobei das eine oder die mehreren Pflaster mit 0,07 mg des Neostigmins pro Kilogramm des Gewichts des Patienten und 0,014 mg des Glycopyrrolats pro Kilogramm des Gewichts des Patienten getränkt sind.

8. Pharmazeutische Zusammensetzung zur Verwendung nach Anspruch 1, wobei das eine oder die mehreren Pflaster mit 0,05 mg Neostigmin pro Kilogramm des Gewichts des Patienten und 0,01 mg Glycopyrrolat pro Kilogramm des Gewichts des Patienten getränkt sind.

9. Pharmazeutische Zusammensetzung zur Verwendung nach Anspruch 1, wobei die Iontophorese die therapeutisch wirksame Menge der Arzneimittelkombination mit 0,1 mg bis 5 mg des Neostigmins pro Minute und 0,1 mg bis 2,5 mg des Glycopyrrolats pro Minute abgibt.

10. Pharmazeutische Zusammensetzung zur Verwendung nach Anspruch 1, wobei die chronische intestinale Pseudoobstruktion das Ergebnis einer oder mehrerer der folgenden Erkrankungen ist: chronische Verstopfung, idiopathische abdominale Distension, abdominale Schmerzen, abdominale Krämpfe, Reizdarmsyndrom, Megakolon assoziiert mit Hyperthyreose, Hypomotilität des Dickdarms assoziiert mit Diabetes mellitus, einer neurologischen Störung, einer myopathischen Störung, einer geriatrischen Hypomotilitätsstörung, ein jejunal-ilealer Bypass mit sekundärem Megakolon, Hypomotilität assoziiert mit Krebs-Chemotherapie, Hypomotilität assoziiert mit Verbrennungen oder Belastungen, Hypomotilität assoziiert mit Depressionen, Parkinson-Krankheit, postoperative intestinale Distension, amyotropher Lateralsklerose, Spina bifida, multipler Sklerose oder Demenz.

11. Pharmazeutische Zusammensetzung zur Verwendung nach Anspruch 1, wobei die Verabreichung mindestens einmal pro Woche über einen Zeitraum von mindestens einem Monat erfolgt.

12. Pharmazeutische Zusammensetzung zur Verwendung nach Anspruch 11, wobei die Verabreichung über einen Zeitraum von mindestens sechs Monaten erfolgt.

13. Pharmazeutische Zusammensetzung zur Verwendung nach Anspruch 1, wobei die Verabreichung mindestens dreimal pro Woche über einen Zeitraum von mindestens einem Monat erfolgt.

## Revendications

1. Composition pharmaceutique comprenant une combinaison de médicaments comprenant de la néostigmine et du glycopyrrolate pour une utilisation dans un procédé de soin de l'intestin, le procédé comprenant l'administration par iontophorèse d'une quantité thérapeutiquement efficace de la composition pharmaceutique à un sujet présentant une pseudo-obstruction intestinale chronique pour soulager une constipation chronique,
dans laquelle le rapport de la néostigmine au glycopyrrolate est de 2,5:1 à 10:1 en poids, et
dans laquelle l'iontophorèse comprend
la mise en place d'un ou plusieurs patchs, collectivement imprégnés avec la quantité thérapeutiquement efficace de la combinaison de médicaments, sur la peau du sujet, dans laquelle chacun des un ou plusieurs patchs est apparié avec un fil électrique, et
la délivrance d'un courant électrique à travers le fil électrique pour que chacun des un ou plusieurs patchs apporte la quantité thérapeutiquement efficace de la combinaison de médicaments à travers la barrière cutanée du sujet dans les vaisseaux sanguins microvasculaires du sujet.

2. Composition pharmaceutique pour une utilisation selon la revendication 1, dans laquelle la quantité thérapeutiquement efficace de la combinaison de médicaments comprend un rapport de la néostigmine au glycopyrrolate d'environ 5:1 en poids.

3. Composition pharmaceutique pour une utilisation selon la revendication 1, dans laquelle la pseudo-obstruction intestinale chronique est un résultat d'une lésion de la moelle épinière, et dans laquelle la lésion de la moelle épinière entraîne une paraplégie ou une quadriplégie.

4. Composition pharmaceutique pour une utilisation selon la revendication 1, dans laquelle la délivrance du courant électrique à travers le fil électrique comprend la délivrance d'un courant positif d'environ 4,0 mA par minute pendant environ 10 minutes.

5. Composition pharmaceutique pour une utilisation selon la revendication 1, dans laquelle les un ou plusieurs patchs sont imprégnés avec jusqu'à 15 mg de la néostigmine et jusqu'à 3 mg du glycopyrrolate.

6. Composition pharmaceutique pour une utilisation selon la revendication 1, dans laquelle les un ou plusieurs patchs sont imprégnés avec jusqu'à 10 mg de la néostigmine et jusqu'à 2 mg du glycopyrrolate.

7. Composition pharmaceutique pour une utilisation selon la revendication 1, dans laquelle les un ou plusieurs patchs sont imprégnés avec 0,07 mg de la néostigmine par kilogramme de poids du sujet et 0,014 mg du glycopyrrolate par kilogramme de poids du sujet.

8. Composition pharmaceutique pour une utilisation selon la revendication 1, dans laquelle les un ou plusieurs patchs sont imprégnés avec 0,05 mg de la néostigmine par kilogramme de poids du sujet et 0,01 mg du glycopyrrolate par kilogramme de poids du sujet.

9. Composition pharmaceutique pour une utilisation selon la revendication 1, dans laquelle l'iontophorèse délivre la quantité thérapeutiquement efficace de la combinaison de médicaments à raison de 0,1 mg à 5 mg de la néostigmine par minute et de 0,1 mg à 2,5 mg du glycopyrrolate par minute.

10. Composition pharmaceutique pour une utilisation selon la revendication 1, dans laquelle la pseudo-obstruction intestinale chronique est un résultat d'un ou plusieurs de la constipation chronique, de la distension abdominale idiopathique, d'une douleur abdominale, de crampes abdominales, du syndrome du côlon irritable, du mégacôlon associé à une hyperthyroïdie, d'une hypomotilité du côlon associée au diabète sucré, d'un trouble neurologique, d'un trouble myopathique, d'un trouble de l'hypomotilité gériatrique, d'une dérivation jéjunale-iléale avec mégacôlon secondaire, d'une hypomotilité associée à une chimiothérapie d'un cancer, d'une hypomotilité associée à des brûlures ou à des stress, d'une hypomotilité associée à une dépression, de la maladie de Parkinson, d'une distension intestinale post-opératoire, de la sclérose latérale amyotrophique, du spina bifida, de la sclérose en plaques ou de la démence.

11. Composition pharmaceutique pour une utilisation selon la revendication 1, dans laquelle l'administration se produit au moins une fois par semaine pendant une période d'au moins un mois.

12. Composition pharmaceutique pour une utilisation selon la revendication 11, dans laquelle l'administration se produit pendant une période d'au moins six mois.

13. Composition pharmaceutique pour une utilisation selon la revendication 1, dans laquelle l'administration se produit au moins trois fois par semaine pendant une période d'au moins un mois.
